# EUROPÄISCHE PATENTSCHRIFT

(11) **EP 0 946 176 B1**
(45) Veröffentlichungstag und Bekanntmachung des Hinweises auf die Patenterteilung: **27.03.2002**
(21) Anmeldenummer: 97954354.3
(22) Anmeldetag: 04.12.1997
(51) Int. Cl.: A61K 31/47, A61P 1/04

(54) **VERWENDUNG VON 7-(2-OXA-5,8-DIAZABICYCLO 4.3.0]NON-8-YL)-CHINOLONCARBONSÄURE- UND -NAPHTHYRIDONCARBONSÄURE-DERIVATEN ZUR HERSTELLUNG EINES ARZNEIMITTELS ZUR THERAPIE VON HELICOBACTER-PYLORI-INFEKTIONEN UND DEN DAMIT ASSOZIIERTEN GASTRODUODENALEN ERKRANKUNGEN**
THE USE OF 7-(2-OXA-5,8-DIAZABICYCLO 4.3.0]NON-8-YL)-QUINOLONE CARBOXYLIC ACID AND NAPHTHYRIDON CARBOXYLIC ACID DERIVATIVES FOR MANUFACTURE OF A MEDICAMENT FOR THE TREATMENT OF HELICOBACTER PYLORI INFECTIONS AND ASSOCIATED GASTRODUODENAL DISEASES
UTILISATION DE DERIVES D'ACIDE 7-(2-OXY-5,8-DIAZABICYCLO 4.3.0]NON-8-YL)QUINOLEONE ET NAPHTYRIDONECARBOXYLIQUE POUR L'OBTENTION D'UN MEDICAMENT DESTINE AU TRAITEMENT D'INFECTIONS PAR HELICOBACTER PYLORI ET DES MALADIES GASTRO-DUODENALES QUI Y SONT ASSOCIEES

(30) Priorität: 16.12.1996 DE 19652239
(43) Veröffentlichungstag der Anmeldung: 06.10.1999
(73) Patentinhaber: BAYER AG, 51368 Leverkusen (DE)
(72) Erfinder: MATZKE, Michael, D-42113 Wuppertal (DE); PETERSEN, Uwe, D-51375 Leverkusen (DE); JAETSCH, Thomas, D-50668 Köln (DE); BARTEL, Stephan, D-51465 Bergisch Gladbach (DE); SCHENKE, Thomas, D-51469 Bergisch Gladbach (DE); HIMMLER, Thomas, D-51519 Odenthal-Glöbusch (DE); BAASNER, Bernd, D-51467 Bergisch Gladbach (DE); WERLING, Hans-Otto, D-42113 Wuppertal (DE); SCHALLER, Klaus, D-42109 Wuppertal (DE); LABISCHINSKI, Harald, D-42113 Wuppertal (DE)
(86) Internationale Anmeldenummer: EP9706781
(87) Internationale Veröffentlichungsnummer: WO9826779

(56) Entgegenhaltungen:
- EP-A- 0 276 700
- EP-A- 0 350 733
- EP-A- 0 550 903
- EP-A- 0 603 887
- EP-A- 0 676 199
- DE-A- 4 329 600
- GB-A- 2 289 674

## Beschreibung

Die Erfindung betrifft die Verwendung von Chinolon- und Naphthyridoncarbonsäure-Derivaten, die in 7-Stellung durch einen 2-Oxa-5,8-diazabicyclo[4.3.0]non-8-yl-Rest substituiert sind, sowie ihre Salze zur Therapie von Helicobacter-pylori-Infektionen und den damit assoziierten gastroduodenalen Erkrankungen.

Mit der Wiederentdeckung von *Helicobacter pylori* (H. pylori; früher Campylobacter pylori) durch Warren und Marshall 1983 konnten in den darauffolgenden Jahren die pathophysiologischen Vorstellungen über die Entstehung gastroduodenaler Erkrankungen des Menschen grundlegend weiterentwickelt werden.

H. pylori gilt als Auslöser der Typ B-Gastritis und scheint eine ursächliche Rolle in der Perpetuierung der peptischen Ulkuserkrankung zu spielen. Epidemiologische und pathologische Untersuchungen weisen ebenfalls auf einen Zusammenhang zwischen Langzeitkolonisierung der Magenmucosa mit dem Bakterium und der Entstehung bestimmter Formen des Magenkarzinoms hin. Deshalb wurde H. pylori im Jahre 1994 zum Karzinogen erster Klasse (gefährlichste Krebsauslöser-Kategorie) eingestuft. Ein seltener Magenkrebs, das MALT-Lymphom (mucosa-associated lymphoid tissue), scheint ebenfalls häufig durch den Keim verursacht zu sein. In ersten Kasuistiken verschwanden tatsächlich nach H. pylori-Eradikation nicht nur die reaktiven Infiltrate, sondern auch ein Teil der niedrig malignen MALT-Lymphome. Auch werden Zusammenhänge mit der Riesenfaltengastritis diskutiert. Die Rolle von H. pylori beim Reizmagen (nichtulzerösen Dyspepsie) ist noch unklar.

Verschiedene epidemiologische Studien kommen zu dem Ergebnis, daß circa die Hälfte der Weltbevölkerung mit dem Bakterium infiziert ist. Die Wahrscheinlichkeit der Besiedlung des Magens mit Helicobacter steigt mit dem Lebensalter. Die optimale Anpassung von Helicobacter an die Lebensbedingungen in dem außergewöhnlichen, konkurrenzarmen Habitat Magen scheint die Voraussetzung für die erfolgreiche Etablierung der chronischen Infektion und für die weite Verbreitung dieser pathogenen Spezies zu sein.

Die Erreger sind mit ihren Geißeln nicht nur im flüssigen Milieu, sondern auch im viskosen Mukus der Magenschleimhaut sehr beweglich, adhärieren an die Magenepithelzellen und vermehren sich am besten bei einem Sauerstoffgehalt von 5 %, wie er im Schleim der Magenwand herrscht. Außerdem bilden die Bakterien große Mengen des Enzyms Urease, das Harnstoff in Ammoniak und Kohlendioxid spaltet. Möglicherweise hilft ihnen die entstehende 'Ammoniakwolke', das saure Milieu in der Mikroumgebung zu neutralisieren und sich so vor der aggressiven Magensäure zu schützen.

### Peptische Ulkuserkrankung

Die Einführung der Histamin-H₂-Rezeptorantagonisten in den 70er Jahren stellte einen Meilenstein in der Therapie der peptischen Ulkuserkrankung dar. Die Häufigkeit chirurgischer Eingriffe zur Behandlung des Ulkusleidens nahm damit weltweit dramatisch ab. Dieses Prinzip der Säureblockade wurde durch die Entwicklung der noch stärker wirksamen Protonenpumpeninhibitoren noch weiter verbessert

Durch die säurehemmende Therapie können allerdings nur die Symptome der Ulkuserkrankung, nicht der natürliche Verlauf der Erkrankung, der durch das Auftreten von Rezidiven gekennzeichnet ist, kausal - sprich durch keimabtötende Behandlung - beeinflußt werden. Denn praktisch alle Ulcus-duodeni-Patienten und die überwiegende Mehrzahl der Patienten mit Ulcus ventriculi weisen eine H. pylori-Infektion des Magens auf und leiden somit an Infektionskrankheiten. Nur Ulzerationen, die durch nichtsteroidale Antiphlogistika hervorgerufen werden, sind nicht mit einer H. pylori-Infektion assoziiert.

Deshalb sollten nach den Empfehlungen einer Konsensus-Konferenz, die im Jahre 1994 von der amerikanischen Gesundheitsbehörde (NIH) veranstaltet wurde, bei positivem Keimnachweis alle Patienten mit peptischen Ulcera einer gegen H. pylori gerichteten Eradikationstherapie zugeführt werden (NIH Consensus Statement 1: 1-23; 1994). Die Argumente dazu lieferten kontrollierte Therapiestudien, in denen gezeigt werden konnte, daß nach erfolgreicher Keimeradikation die Ulkusrezidivraten drastisch sinken ( 0% - 29% versus 61% - 95%).

### H. pylori-Therapie

Die derzeitige Eradikation des H. pylori gestaltet sich in der Praxis problematisch. Eine einfache und dennoch verläßlich wirksame Therapie steht nicht zur Verfügung. Der Keim findet sich gut geschützt und schwer angreifbar unter der Mukusschicht.

H. pylori ist in vitro gegenüber zahlreichen Antibiotika empfindlich. Diese sind allerdings in vivo als Monotherapie nicht effektiv. Dazu zählen u. a. Penicillin, Amoxicillin, Tetracyclin, Erythromycin, Ciprofloxacin, Metronidazol und Clarithromycin. Auch Wismutsalze und im geringen Maße sogar Protonenpumpeninhibitoren (Omeprazol, Lansoprazol) sind in vitro, nicht aber in vivo antibakteriell wirksam.

Unter allen bisher zur Eradikation von H. pylori angewandten Therapiemodalitäten sind derzeit nur die folgenden Tripel-Therapien ausreichend wirksam:
1. klassische Wismut-Tripel-Therapie (Wismutsalz plus zwei Antibiotika) und die
2. modifizierte Tripel-Therapie (Säurehemmer plus zwei Antibiotika).

Allerdings sind diese Regime umständliche Eradikationsverfahren mit schlechter Compliance, die bis zu 35% mit Nebenwirkungen (Bauchschmerzen, Übelkeit, Durchfall, Mundtrockenheit, Geschmacksstörungen und allergische Hautreaktionen etc.) behaftet sein können. Deshalb wird eine breite Anwendung erschwert. Ein weiterer großer Nachteil ist die hohe Zahl der täglich einzunehmenden Medikamente (12-16 Tabletten/Tag). Dies wird besonders deutlich bei der Quadrupel-Therapie, bei der gleichzeitig zur klassischen Tripel-Therapie ein Säuresekretionshemmer verabreicht wird.

Die in Deutschland propagierte, besser verträgliche Dual-Therapie (Kombination von Amoxicillin mit Omeprazol) ist allerdings nur gering wirksam und scheint bei mit Omeprazol vorbehandelten Patienten und bei Rauchern sogar weitgehend zu versagen.

Bei den Tripel-Therapien werden als antibiotische Komponente in der Regel Amoxicillin, Nitroimidazolverbindungen (Metronidazol, Tinidazol), Tetracyclin sowie neuerdings Makrolide (Clarithromycin) [in 3-4 Teildosen] verabreicht.

Weltweit werden Eradikationsraten von 70 - 90% erreicht. Verschiedene Faktoren können diesen Eradikationserfolg allerdings beeinflussen:
1. An erster Stelle ist die Resistenz des Keimes (Entwicklungsländer: bis zu 60%, Deutschland: bis zu 10%) gegenüber Metronidazol, dem am häufigsten in der Tripel-Therapie eingesetzten Antibiotikum, zu nennen. Auch bei der Behandlung mit Clarithromycin wird auf den Nachteil einer Resistenzentwicklung von bis zu 10% hingewiesen.
2. Als ein weiterer Faktor ist die oben erwähnte Compliance der Patienten zu nennen.

### Tiermodell

Als ein geeignetes Tiermodell wurde ein H. felis-Mausmodell beschrieben [A. Lee et al., Gastroentrology **99:** 1315-1323 (1990)] und von uns so modifiziert, daß es sich für das Screening und die vergleichende Bewertung o. g. Verbindungen sehr gut eignet.

Das korkenzieherähnliche, ureasebildende Bakterium H. felis ist trotz großer morphologischer Unterschiede mit H. pylori sehr nah verwandt. H. felis ist ein natürlicher Bewohner der Magenmucosa von Hunden und Katzen. Nach oraler Inokulation kolonisieren die Erreger auch den Mäusemagen in ähnlicher Weise wie H. pylori den Magen des Menschen. Die etablierte chronische Langzeitinfektion führt bei Mäusen zur aktiven Gastritis und induziert eine entsprechende Immunantwort.

Die im H. felis-Mausmodell ermittelte therapeutische Effektivität von Prüfpräparaten wird in der Literatur als prädiktiv für die entsprechende klinische Wirksamkeit angesehen.

Trotz sehr guter In-vitro-Aktivität von Antibiotika (z. B. Amoxicillin oder Erythromycin) gegen H. pylori, zeigen diese nach monotherapeutischer Anwendung klinisch keine signifikante therapeutische Wirkung. Diese Tatsache wird auch duch das H. felis-Mausmodell wiedergegeben. Entsprechend konnte die klinisch anerkannte eradikative Wirkung der klassischen Tripel-Therapie auch im H. felis Mausmodell bestätigt werden.

Aus den EP-A-350733 und EP-A-550 903 (Bayer) sind bereits antibakteriell wirksame 7-(2-Oxa-5,8-diazabicyclo[4.3.0]non-8-yl)-chinolon- und naphthyridoncarbonsäure-Derivate bekannt geworden. In der JP 8048629 (Dainippon) wurde beschrieben, daß Verbindungen wie 8-Chlor-1-cyclopropyl-7-([S,S]-2,8-diazabicyclo[4.3.0]non-8-yl)-6-fluor-1,4-dihydro-4-oxo-3-chinolincarbonsäure (BAY Y 3118) eine antibakterielle Wirkung gegenüber H. pylori aufweisen. Es ist auch bekannt, daß eine Reihe von hochwirksamen Chinolonen, wie zum Beipiel Ciprofloxacin, Lomefloxacin oder Ofloxacin (Journal of Antimicrobial Chemotherapy **22,** 631-636 [1988], Antimicrobial Agents and Chemotherapy, **33**, 108-109 [1989]), in vitro gegenüber Helicobacter spp. eine Wirkung aufweisen. Es zeigte sich jedoch im Tiermodell (Helicobacter felis, Maus), daß diese klinisch eingesetzten antibakteriell wirksamen Chinolone in therapeutisch verwendeten Dosen nicht in der Lage sind, zu einer Eradikation des Keimes zu führen. Auch durch eine mono-therapeutische Behandlung mit hochwirksamen Chinolonen, die bisher nicht in den Markt eingeführt wurden, wie zum Beispiel mit dem bereits erwähnten BAY Y 3118, kann keine Eradikation von H. felis im Mausmodell erreicht werden, ohne daß nicht auf Grund der Toxizität der Verbindung zumeist ein großer Teil der Tiere stirbt. Die Verwendung von Trovafloxacin oder seinen Derivaten in Kombination mit anderen Antibiotika wie Amoxicillin oder Tetracyclinen oder Protonenpumpeninhibitoren wie Omeprazol zur Therapie von H. pylori wird in EP-A-0 676 199 und GB-A-2 289 674 (Pfizer) beschrieben.

Die (1) EP-A 603 887 betrifft Chinolonderivat-Wirkstoffe, die zur Bekämpfung von Campylobacter sp. verwendet werden können. Helicobacter pylori wird in der Druckschrift nicht erwähnt.

In der (2) EP-A 676 199 werden Chinolonderivate zur Bekämpfung von Helicobacter pylori beschrieben. Die dort beschriebenen Wirkstoffe unterscheiden sich von denen der vorliegenden Erfindung in der Art des aminischen Substituenten in 7-Position. Gegenüber diesen Verbindungen zeigen die erfindungsgemäß zu verwendenden Wirkstoffe weniger unerwünschte Nebenwirkungen.

Die der Erfindung zugrundeliegenden Aufgabe war daher, relativ gut verträgliche Wirkstoffe zu finden, die in der Lage sind, dieses hochspezialisierte Bakterium durch eine einfache Monotherapie zu eradizieren.

Es wurde nun gefunden daß Verbindungen der allgemeinen Formel (I) 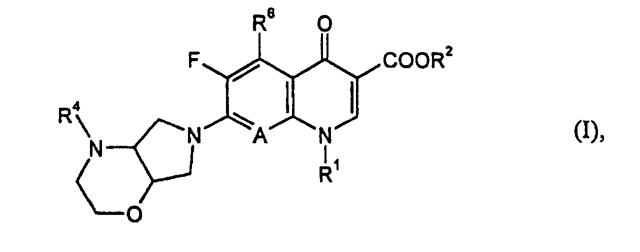 in welcher
- R¹: für gegebenenfalls durch Halogen ein- oder zweifach substituiertes Alkyl mit 1 bis 4 C-Atomen, gegebenenfalls durch 1 oder 2 Fluoratome substituiertes Phenyl oder gegebenenfalls durch 1 oder 2 Fluoratome substituiertes Cyclopropyl steht,
- R²: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- A: für N oder C-R³ steht, wobei
R³ für Wasserstoff, Halogen, Methyl, Methoxy, Difluormethoxy oder Cyano steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verbunden ist, bilden kann,
- R⁴: für Wasserstoff, Benzyl, C₁-C₃-Alkyl, (5-Methyl-2-oxo-1,3-dioxol-4-yl)-methyl, Reste der Strukturen -CH=CH-COOR⁵, -CH₂CH₂COOR⁵, -CH₂CH₂CN, -CH₂CH₂COCH₃, -CH₂COCH₃, in denen
R⁵ fiir Methyl oder Ethyl,
- R⁶: für Wasserstoff, Amino, Hydroxy, Methyl oder Halogen steht,
in Form von Racematen, Diastereomerengemischen oder als enantiomerenreine oder diastereomerenreine Verbindungen, deren pharmazeutisch verwendbare Hydrate und/oder Salze, wie Säureadditionssalze sowie die Alkali-, Erdalkali-, Silber- und Guanidiniumsalze der zugrundeliegenden Carbonsäuren, eine hohe antibakterielle Wirkung gegenüber Helicobacter spp. aufweisen und zur Eradikation dieses Erregers verwendet werden können.

Bevorzugt sind Verbindungen der Formel (I), in welcher
- R¹: für gegebenenfalls durch Fluor ein- oder zweifach substituiertes tert.-Butyl oder gegebenenfalls durch 1 Fluoratom substituiertes Cyclopropyl steht,
- R²: für Wasserstoff, Alkyl mit 1 bis 4 Kohlenstoffatomen oder (5-Methyl-2-oxo-1,3-dioxol-4-yl)-methyl steht,
- A: für C-R³ steht, wobei
R³ für Wasserstoff, Fluor, Methoxy, Difluormethoxy, Cyano steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verbunden ist, bilden kann,
- R⁴: für Wasserstoff, C₁-C₃-Alkyl, Reste der Strukturen, -CH₂CH₂COOR⁵, -CH₂CH₂CN, -CH₂COCH₃, in denen
R⁵ für Methyl oder Ethyl steht,
- R⁶: für Wasserstoff, Amino oder Methyl steht,
und deren pharmazeutisch verwendbare Hydrate und/oder Salze, wie Säureadditionssalze sowie die Alkali-, Erdalkali-, Silber- und Guanidiniumsalze der zugrundeliegenden Carbonsäuren.

Besonders bevorzugt sind Verbindungen der Formel (I), in welcher
- R¹: für gegebenenfalls durch Fluor ein- oder zweifach substituiertes tert.-Butyl oder Cyclopropyl steht,
- R²: für Wasserstoff, Methyl oder Ethyl steht,
- A: für C-R³ steht, wobei
R³ für Wasserstoff, Methoxy, Difluormethoxy, Cyano steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verbunden ist, bilden kann,
- R⁴: für Wasserstoff oder Methyl steht,
- R⁶: für Wasserstoff steht,
und deren pharmazeutisch verwendbare Hydrate und/oder Salze, wie Säureadditionssalze sowie die Alkali-, Erdalkali-, Silber- und Guanidiniumsalze der zugrundeliegenden Carbonsäuren.

Gegenstand der vorliegenden Erfindung sind auch die neuen Verbindungen 8-Cyano-1-cyclopropyl-6-fluor-7-(2-oxa-5,8-diazabicyclo[4.3.0]non-8-yl)-1,4-dihydro-4-oxo-3-chinolincarbonsäure und 1-Cyclopropyl-8-difluormethoxy-6-fluor-1,4-dihydro-7-(2-oxa-5,8-diazabicyclo[4.3.0]non-8-yl)-4-oxo-3-chinolincarbonsäure, insbesondere in diastereomerenreiner und enantiomerenreiner Form und deren pharmazeutisch verwendbare Hydrate und/oder Salze, wie Säureadditionssalze sowie die Alkali-, Erdalkali-, Silber- und Guanidiniumsalze der zugrundeliegenden Carbonsäuren. Besonders bevorzugt ist die 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure.

Die Verbindungen, die für die erfindungsgemäße Verwendung geeignet sind, sind zum Teil bereits aus EP-A-0 350 733, EP-A-0 550 903 sowie aus DE-A-4 329 600 bekannt oder können nach den dort beschriebenen Verfahren hergestellt werden.

Verwendet man beispielsweise 9,10-Difluor-3,8-dimethyl-7-oxo-2,3-dihydro-7H-pyrido-[1,2,3-d,e][1,3,4]benzoxadiazin-6-carbonsäure und 2-Oxa-5,8-diazabicyclo-[4.3.0]nonan, so kann der Reaktionsverlauf durch das folgende Formelschema wiedergegeben werden:

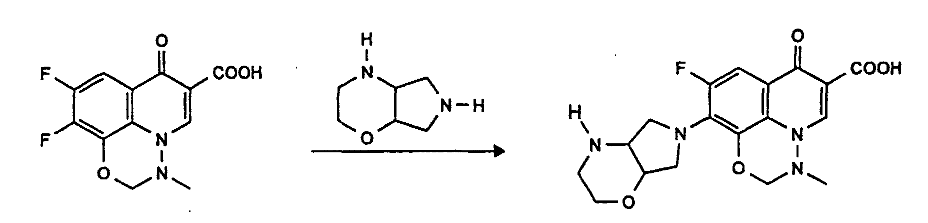

Die zur Herstellung der erfindungsgemäßen Verbindungen der Fomel (I) eingesetzten 7-Halogen-chinoloncarbonsäurederivate sind bekannt oder können nach bekannten Methoden hergestellt werden. So sind die 7-Chlor-8-cyano-1-cyclopropyl-6-fluor-1,4-dihydro-4-oxo-3-chinolincarbonsäure beziehungsweise der 7-Chlor-8-cyano-1-cyclopropyl-6-fluor-1,4-dihydro-4-oxo-3-chinolincarbonsäure-ethylester in der EP-A-0 276 700 beschrieben worden. Die entsprechenden 7-Fluorderivate lassen sich beispielsweise auch über die folgende Reaktionsfolge aufbauen:

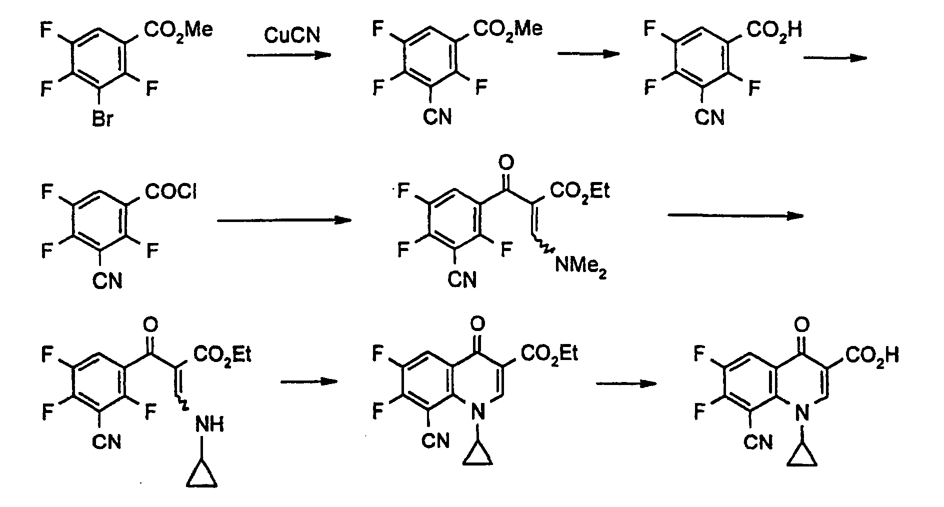

Ein Alternativverfahren zur Herstellung der Zwischenverbindung 2,4-Dichlor-3-cyano-5-fluor-benzoylchlorid, das als Ausgangsprodukt zur Herstellung von 7-Chlor-8-cyano-1-cyclopropyl-6-fluor-1,4-dihydro-4-oxo-3-chinolincarbonsäure dient (EP-A-0 276 700) und das in 3-Cyano-2,4,5-trifluor-benzoylfluorid überführt werden kann, geht von 5-Fluor-1,3-xylol aus: 5-Fluor-1,3-xylol wird in Gegenwart eines Katalysators unter ionischen Bedingungen im Kern zweifach zum 2,4-Dichlor-5-fluor-1,3-dimethylbenzol chloriert und dieses anschließend unter radikalischen Bedingungen in den Seitenketten zu 2,4-Dichlor-5-fluor-3-dichlormethyl-1-trichlormethylbenzol chloriert. Dieses wird über die 2,4-Dichlor-5-fluor-3-dichlormethylbenzoesäure zur 2,4-Dichlor-5-fluor-3-formyl-benzoesäure verseift und anschließend zur 2,4-Dichlor-5-fluor-3-*N*-hydroxyiminomethyl-benzoesäure umgesetzt. Durch Behandlung mit Thionylchlorid wird 2,4-Dichlor-3-cyano-5-fluor-benzoylchlorid erhalten, welches noch durch einen Chlor/Fluor-Austausch zu 3-Cyano-2,4,5-trifluor-benzoylfluorid umgesetzt werden kann.

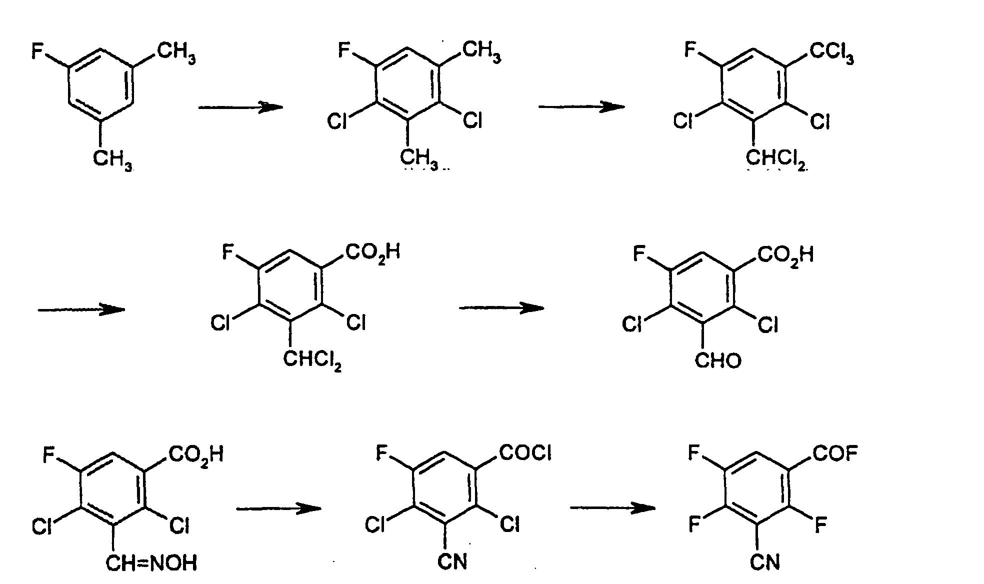

Die zur Herstellung der erfindungsgemäßen Verbindungen der Formel (I) eingesetzten Amine sind aus EP-A-0 550 903, EP-A-0 551 653 sowie aus DE-A-4 309 964 bekannt.

Eine Alternative zur Synthese von 1S,6S-2-Oxa-5,8-diazabicyclo[4.3.0]nonan-Dihydrobrornid beziehungsweise der freien Base 1S,6S-2-Oxa-5,8-diazabicyclo[4.3.0]nonan sowie der entsprechenden 1R,6R-Enantiomeren stellt der folgende Weg dar:

Ausgangsprodukt für diese Synthese ist das cis-1,4-Dihydroxy-2-buten, das nach Mesylierung zum Bis-mesylat mit Tosylamid zum 1-Tosylpyrrolidin umgesetzt wird.

Dieses wird m-Chlorperbenzoesäure ins Epoxid überführt. Die Ringöffnung des Epoxids erfolgt durch Erhitzen mit Ethanolamin in Isopropanol zum trans-3-Hydroxy-4-(2-hydroxy-ethylamino)-1-(toluol-4-sulfonyl)-pyrrolidin in über 80%-iger Ausbeute. Anschließend wird in Pyridin/Tetrahydrofuran mit Tosylchlorid unter Kühlung zum Tris-Tosylat umgesetzt, das als Rohprodukt im Gemisch mit etwas Tetra-Tosyl-Derivat unter basichen Reaktionsbedingungen zum racemischen trans-5,8-Bis-tosyl-2-oxa-5,6-diazabicyclo[4.3.0]nonan cylisiert wird. Auf dieser Stufe erfolgt mit hoher Selektivität eine chromatographische Racematspaltung an kieselgelgebundenem Poly(N-meth-acryloyl-L-leucin-d-menthylamid) als stationärer Phase. Das gewünschte Enantiomer, (1S,6S)-5,8-Bis-tosyl-2-oxa-5,6-diazabicyclo[4.3.0]nonan, wird mit einer Reinheit von > 99 % ee isoliert. Die Abspaltung der p-Tosyl-Schutzgruppen erfolgt mit HBr-Eisessig zum 1S,6S-2-Oxa-5,8-diazabicyclo[4.3.0]nonan-Dihydrobromid, das mit Basen wie zum Beispiel Natrium- oder Kaliumhydroxid oder mit Hilfe eines Ionenaustauschers in die freie Base überführt werden kann. Die analoge Reaktionsfolge kann auch zur Herstellung von 1R,6R-2-Oxa-5,8-diazabicyclo[4.3.0]nonan-Dihydrobromid benutzt werden.

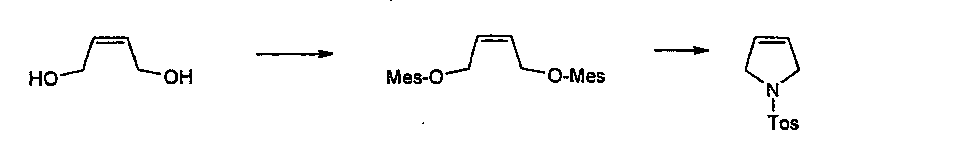

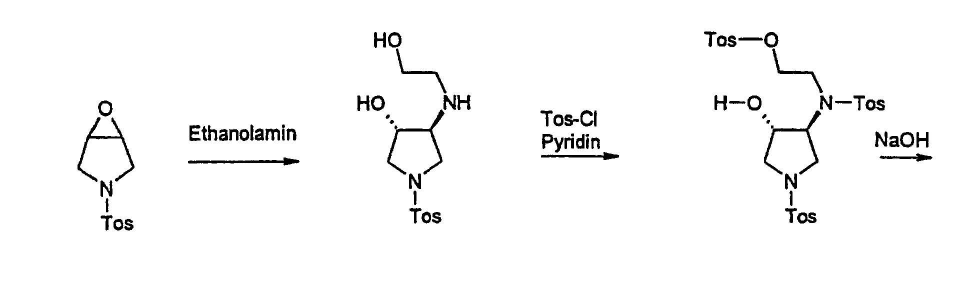

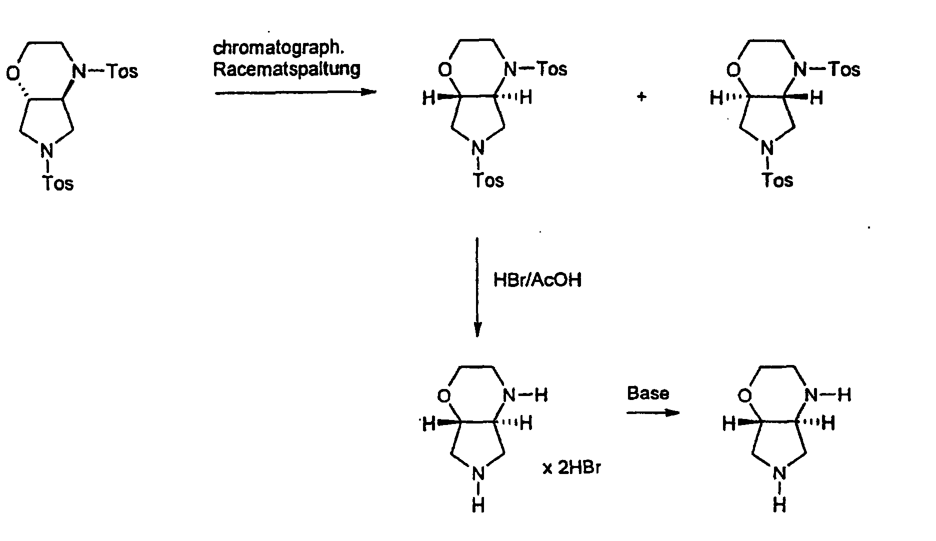

### Synthese von 1S,6S-2-Oxa-5,8-diazabicyclo[4.3.0]nonan

Als Beispiele für erfindungsgemäße Verbindungen seien außer den in den Herstellungsbeispielen aufgeführten Verbindungen die in folgender Tabelle 1 aufgeführten Verbindungen genannt, die sowohl in racemischer Form als auch als enantiomerenreine beziehungsweise diastereomerenreine Verbindungen verwendet werden können.

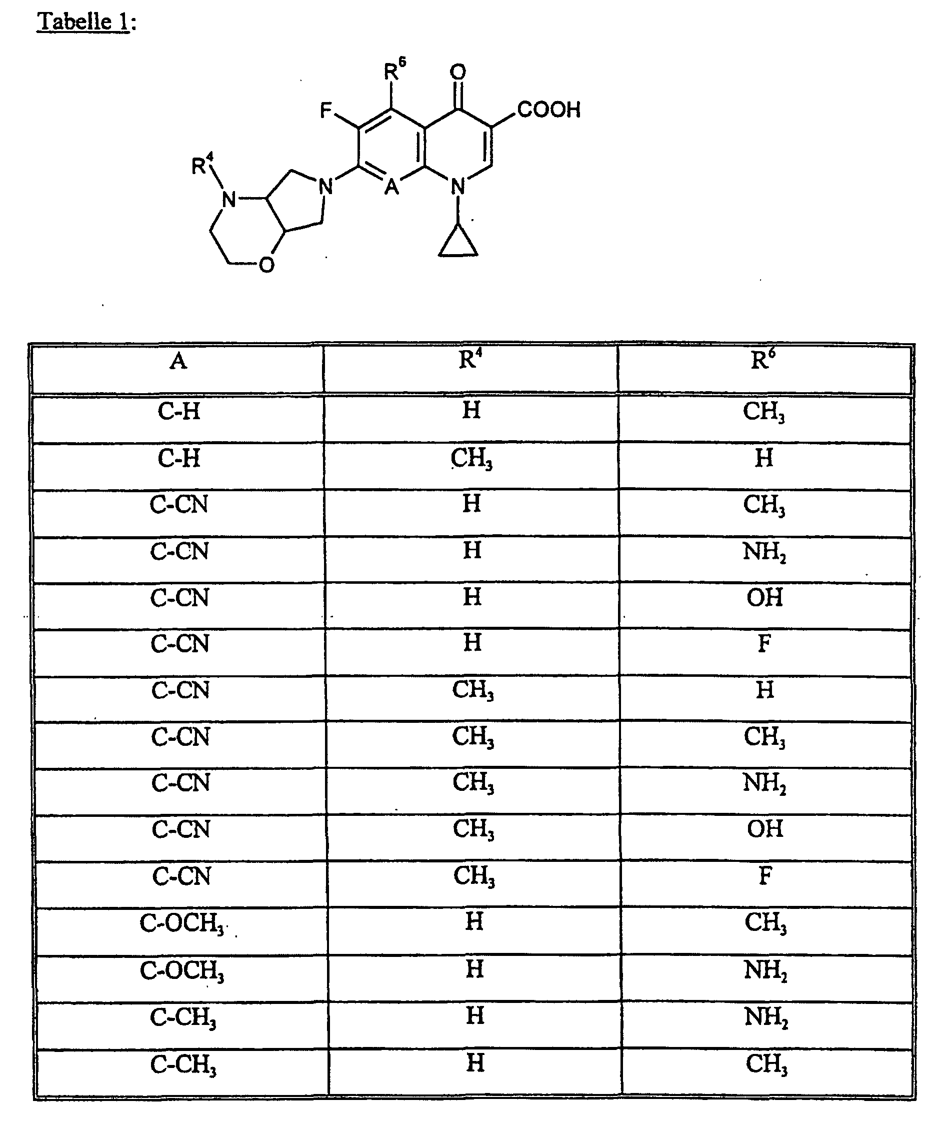

Die erfindungsgemäßen Verbindungen können in Form ihrer Betaine oder in Form der Salze mit einem bis zwei Mol Wasser kristallisieren.

Die erfindungsgemäßen Verbindungen wirken stark antibiotisch und zeigen bei geringer Toxizität ein breites antibakterielles Spektrum gegenüber grampositiven und gramnegativen Keimen, vor allem aber auch gegenüber Helicobacter spp.

Diese wertvollen Eigenschaften ermöglichen ihre Verwendung als chemotherapeutische Wirkstoffe zur Therapie von Helicobacter-pylori-Infektionen und den damit assoziierten gastroduodenalen Erkrankungen, die durch die erfindungsgemäßen Verbindungen verhindert, gebessert und/oder geheilt werden können.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Lösungen, Suspensionen und Emulsionen genannt.

Obwohl die erfindungsgemäßen Verbindungen als monotherapeutische Mittel eingesetzt werden, können sie bei Bedarf auch in Kombination mit anderen Therapeutika verwendet werden. Beispielhaft seien als Kombinationspartner genannt: Nitroimidazolderivate, zum Beispiel Metronidazol; Protonenpumpeninhibitoren, zum Beispiel Omeprazol, Pantoprazol oder Lansoprazol; H₂-Rezeptorantagonisten, wie zum Beispiel Cimetidin, Ranitidin, Famotidin oder Nizatidin; Wismutverbindungen, wie zum Beispiel Wismutsalicylat oder CBS (colloidales Bismut Subcitrat); andere Antibiotika, wie zum Beispiel Amoxicillin, Azlocillin oder Clarithromycin; Antacida.

Die minimalen Hemmkonzentrationen (MHK), die in Tabelle 2 für einige der erfindungsgemäßen Verbindungen beispielhaft im Vergleich zu Ciprofloxacin aufgeführt sind, wurden im Agar-Verdünnungstest auf Columbia-Agar beziehungsweise Basis 2 Agar (Oxoid) mit 10 % lysiertem Pferdeblut entweder bei pH 7 oder pH 5 mit 1 g/l Harnstoff bestimmt. Die Prüfsubstanzen wurden in Replica-Schalen getestet, die bei jeweils doppelter Verdünnung abfallende Konzentrationen des Wirkstoffes enthielten. Zum Beimpfen wurden frische Helicobacter-Kulturen aus Flüssigkultur oder Suspension der Keime von Agarplatten verwandt. Die beimpften Agarplatten wurden bei 37°C in einer Atmosphäre mit 5-10 % CO₂ während 48-72 Stunden bebrütet Der abgelesene MHK-Wert (mg/l) gibt die geringste Wirkstoffkonzentration an, bei der mit bloßem Auge kein Wachstum zu erkennen war. Die folgenden Helicobacter-Isolate wurden verwendet: H. felis ATCC 49179, H. pylori NCTC 11637, H. pylori-Klinikisolat 008.

**Tabelle 2:**

| MHK-Werte (mg/l) einiger erfindungsgemäßer Verbindungen (Agarverdünnungstest) | | |
|---|---|---|
| Beispiel | MHK(mg/l) | |
| | H. pylori 008 | H. pylori 11637 |
| 1A | 0,06 | n.d. |
| 2 | 0,06 | n.d. |
| 4 | 0,25 | 0,06 |
| 6 | 0,06 | 0,06 |
| 8 | 0,06 | 0,06 |
| 13 | 0,125 | 0,06 |
| Ciprofloxacin | 0,125 | 0,125 |

Für Untersuchungen im Tiermodell wurden weibliche Swiss Mäuse (8 bis 12 Wochen alt, SPF-Zucht) mit handelsüblichem Futter und Wasser gehalten. Zur Kolonisation wurde ein definierter H.-felis-Stamm (ATCC 49179) verwendet Die Bakterien werden als Suspension (0,1 ml mit 10⁸-10⁹ Bakterien) 4-mal innerhalb von 7 Tagen per Schlundsonde appliziert. Alternativ hierzu wurden zur Infektion auch Magenhomogenate von vorher infizierten Mäusen verwendet.

3-5 Tage nach Etablierung der Infektion wurde die Behandlung, mit Prüfpräparaten begonnen. Als erster Behandlungserfolg wurde die Keimreduktion als "Clearance" 24 Stunden nach der letzten Behandlung (zum Beispiel 3, 7, 10, 14 Tage; 1-3-mal täglich) bestimmt. In einigen Fällen wurde auch die Keim-Eradikation 2-4 Wochen nach Behandlungsende ermittelt. In Anlehnung an den in der klinischen Diagnostik verwendeten "CLO"-Test wurde ein Urease-Test auf Mikrotiter-Basis eingesetzt. Geprüft wurden definierte Magenbiopsate auf Farbumschlag innerhalb 24 Stunden.

In Tabelle 3 ist als Beispiel für die überraschend hohe In-vivo-Wirkung der erfindungsgemäßen Verbindungen der Therapieerfolg nach 7-tägiger Behandlung infizierter Mäuse mit 8-Cyano-1-cyclopropyl-6-fluor-7-((1S,6S)-2-oxa-5,8-diazabicyclo[4.3.0]-non-8-yl)-1,4-dihydro-4-oxo-3-chinolincarbonsäure (Beispiel 1A) sowie für 9-Fluor-3-methyl-10-((1S,6S)-2-oxa-5,8-diazabicyclo[4.3.0]non-8-yl)-7-oxo-2,3-dihydro-7H-pyrido[1,2,3-d,e][1,3,4]benzoxadiazin-6-carbonsäure (Beispiel 2) im Vergleich zur Behandlung mit Ciprofloxacin aufgeführt: Während mit Ciprofloxacin unter diesen Versuchsbedingungen keine Clearance erreicht wird, beträgt diese bei den erfindungsgemäßen Verbindungen 100 %. Eine 10-tägige Behandlung der Mäuse mit 2 x 10mg/kg 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure führte sogar zu einer Eradikation des Keimes.

**Tabelle 3:**

| Therapieerfolg nach 7-tägiger Behandlung von infizierten (H. felis ATCC 49179) Mäusen (5 Tiere pro Gruppe) | | | |
|---|---|---|---|
| Beispiel | Dosis [mg/kg] | Clearance | % |
| 1A | 2 x 10 | 5/5 | 100 |
| 2 | 2 x 10 | 5/5 | 100 |
| Ciprofloxacin | 2 x 10 | 0/5 | 0 |

### Beispiele

### Herstellung der Zwischenprodukte:

### Beispiel Z 1

### 8-Cyano-1-cyclopropyl-6,7-difluor-1,4-dihydro-4-oxo-3-chinolin-carbonsäureethylester

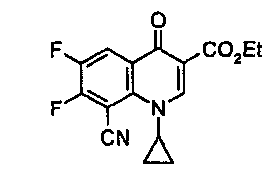

a. 3-Brom-2,4,5-trifluor-benzoesäuremethylester:
   Zu einer Mischung aus 1460 ml Methanol und 340 g Triethylamin werden unter Eiskühlung 772 g 3-Brom-2,4,5-trifluor-benzoylfluorid getropft Es wird 1 Stunde bei Raumtemperatur nachgerührt. Das Reaktionsgemsich wird eingeengt, der Rückstand in Wasser und Methylenchlorid aufgenommen und die wäßrige Phase nochmals mit Methylenchlorid ausgeschüttelt. Nach Trocknen der organischen Phase über Natriumsulfat wird eingeengt und der Rückstand im Vakuum destilliert. Man erhält 752,4 g 3-Brom-2,4,5-trifluorbenzoesäuremethylester vom Siedepunkt 122°C/20 mbar.
b. 3-Cyano-2,4,5-trifluor-benzoesäuremethylester
   269 g 3-Brom-2,4,5-trifluor-benzoesäuremethylester und 108 g Kupfercyanid werden in 400 ml Dimethylformamid während 5 Stunden zum Rückfluß erhitzt. Anschließend werden im Vakuum alle flüchtigen Bestandteile des Reaktionsgemisches abdestilliert. Das Destillat wird dann an einer Kolonne fraktioniert. Man erhält 133 g 3-Cyano-2,4,5-trifluor-benzoesäuremethylester vom Siedepunkt 88-89°C/0,01 mbar.
c. 3-Cyano-2,4,5-trifluor-benzoesäure:
   Eine Lösung von 156 g 3-Cyano-2,4,5-trifluor-benzoesäuremethylester in 960 ml Eisessig, 140 ml Wasser und 69 ml konzentrierter Schwefelsäure wird 8 Stunden zum Rückfluß erhitzt. Anschließend wird die Essigsäure im Vakuum weitgehend abdestilliert und der Rückstand mit Wasser versetzt. Der ausgefallene Feststoff wird abgesaugt, mit Wasser gewaschen und getrocknet. Man erhält 118,6 g 3-Cyano-2,4,5-trifluor-benzoesäure als weißen Feststoff vom Schmelzpunkt 187-190°C.
d. 3-Cyano-2,4,5-trifluor-benzoesäurechlorid:
   111 g 3-Cyano-2,4,5-trifluor-benzoesäure und 84 g Oxalylchlorid werden in 930 ml trockenem Methylenchlorid unter Zusatz einiger Tropfen Dimethylformamid 5 Stunden bei Raumtemperatur gerührt. Anschließend wird das Methylenchlorid abgezogen und der Rückstand im Vakuum destilliert. Man erhält 117,6 g 3-Cyano-2,4,5-trifluor-benzoylchlorid als gelbes Öl.
e. 2-(3-Cyano-2,4,5-trifluor-benzoyl)-3-dimethylamino-acrylsäureethylester:
   Zu einer Lösung von 36,5 g 3-Dimethylamino-acrylsäureethylester und 26,5 g Triethylamin in 140 ml Toluol wird eine Lösung von 55 g 3-Cyano-2,4,5-trifluor-benzoesäurechlorid in 50 ml Toluol so zugetropft, daß die Temperatur zwischen 50 und 55°C bleibt. Dann wird noch 2 Stunden bei 50°C nachgerührt. Das Reaktionsgemisch wird im Vakuum eingeengt und ohne weitere Aufarbeitung in die nächste Stufe eingesetzt.
f. 2-(3-Cyano-2,4,5-trifluor-benzoyl)-3-cyclopropylamino-acrylsäureethylester:
   Zu dem Reaktionsprodukt aus der Stufe e werden bei 20°C 30 g Eisessig getropft. Anschließend wird eine Lösung von 15,75 g Cyclopropylamin in 30 ml Toluol zugetropft. Das Gemisch wird 1 Stunde bei 30°C gerührt. Dann gibt man 200 ml Wasser hinzu, verrührt 15 Minuten, trennt die organische Phase ab und schüttelt sie nochmals mit 100 ml Wasser aus. Dann wird die organische Phase über Natriumsulfat getrocknet und im Vakuum eingeengt. Das so erhaltene Rohprodukt wird ohne weitere Aufarbeitung in die nächste Stufe eingesetzt.
g. 8-Cyano-1-cyclopropyl-6,7-difluor-1,4-dihydro-4-oxo-3-chinolincarbonsäureethylester:
   Das Reaktionsprodukt aus der Stufe fund 27,6 g Kaliumcarbonat werden in 80 ml Dimethylformamid 16 Stunden bei Raumtemperatur gerührt. Das Reaktionsgemisch wird dann in 750 ml Eiswasser gegeben, der Feststoff abgesaugt und mit 80 ml kaltem Methanol gewaschen. Nach Trocknen erhält man 47 g 8-Cyano-1-cyclopropyl-6,7-difluor-1,4-dihydro-4-oxo-3-chinolincarbonsäureethylester vom Schmelzpunkt 209-211°C.

### Beispiel Z 2

### 2,4-Dichlor-5-fluor-1,3-dimethylbenzol

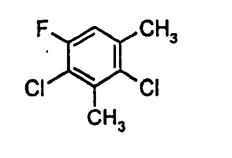

### a) lösungsmittelfrei

In 124 g 3,5-Dimethyl-fluorbenzol werden 1 g wasserfreies Eisen(III)-chlorid vorgelegt und mit der Geschwindigkeit Chlor eingeleitet (ca. 4 h), mit der die Reaktion abläuft. Diese ist anfangs etwas exotherm (Temperaturanstieg von 24 auf 32°C) und wird durch Kühlung unter 30°C gehalten. Nach Zufuhr von 120 g Chlor wird der Ansatz fest. Nach GC-Analyse sind 33,4 % Monochlorverbindung, 58,4 % gewünschtes Produkt und 5 % überchlorierte Verbindungen entstanden. Der Chlorwasserstoff wird abgezogen und die Reaktionsmischung anschließend im Wasserstrahlvakuum an einer Kolonne destilliert:

Im Vorlauf werden bei 72-74°C/22 mbar 49 g 2-Chlor-5-fluor-1,3-dimethylbenzol erhalten. Nach einem Zwischenlauf von 5 g gehen bei 105°C/22 mbar 75 g 2,4-Dichlor-5-fluor-1,3-dimethylbenzol über; Schmelzbereich: 64 - 65°C.

### b) in 1,2-Dichlorethan

1 kg 3,5-Dimethyl-fluorbenzol und 15 g wasserfreies Eisen(III)-chlorid werden in 11 1,2-Dichlorethan vorgelegt und in dem Maße Chlor eingeleitet, wie die Reaktion fortschreitet (ca. 4 h). Die Reaktion ist anfangs exotherm (Temperaturanstieg von 24 auf 32°C) und wird durch Kühlung unter 30°C gehalten. Nach dem Einleiten von 1200 g Chlor sind laut GC-Analyse 4 % Monochlorverbindung, 81,1 % gewünschtes Produkt und 13,3 % überchlorierte Verbindungen entstanden. Nach Abdestillation des Lösungsmittels und des Chlorwasserstoffs wird im Wasserstrahlvakuum an einer Kolonne destilliert:

Im Vorlauf werden 40 g 2-Chlor-5-fluor-1,3-dimethylbenzol erhalten. Nach wenig Zwischenlauf gehen bei 127 - 128°C/50 mbar 1115 g 2,4-Dichlor-5-fluor-1,3-dimethylbenzol über.

### Beispiel Z 3

### 2,4-Dichlor-5-fluor-3-dichlormethyl-1-trichlormethylbenzol

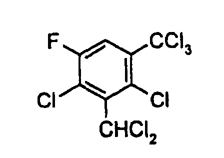

In einer Photochlorierungsapparatur mit Chloreinleitung und Ableitung für den Chlorwasserstoff zu einem Wäscher sowie einer Lichtquelle in der Nähe des Chloreinleitungsrohrs werden 1890 g 2,4-Dichlor-5-fluor-1,3-dimethylbenzol vorgelegt und bei 140 bis 150°C Chlor eindosiert. Innerhalb 30 h werden 3850 g Chlor eingeleitet. Der Gehalt an gewünschtem Produkt beträgt nach GC-Analyse 71,1 %; der Anteil an minderchlorierten Verbindungen 27,7 %.

Die Destillaton über eine 60 cm-Kolonne mit Wilson-Spiralen liefert einen Vorlauf von 1142 g, der erneut in die Chlorierung eingesetzt werden kann. Der Hauptlauf bei 160-168°C/0,2 mbar liefert 2200 g 2,4-Dichlor-5-fluor-3-dichlormethyl-1-trichlormethylbenzol mit einem Schmelzbereich von 74-76°C. Nach Umkristallisieren einer Probe aus Methanol beträgt der Schmelzpunkt 81-82°C.

### Beispiel Z 4

### 2,4-Dichlor-5-fluor-3-formyl-benzoesäure

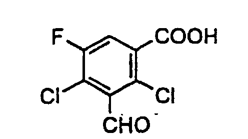

In einer Rührapparatur mit Gasableitung werden 2500 ml 95%-ige Schwefelsäure bei 70°C vorgelegt und unter Rühren 500 g aufgeschmolzenes 2,4-Dichlor-5-fluor-3-dichlormethyl-1-trichlormethylbenzol zugetropft. Es setzt nach kurzer Zeit Chlorwasserstoffentwicklung ein. Man dosiert während 2 h ein und rührt bis zum Ende der Gasentwicklung nach. Nach Abkühlen auf 20°C wird der Ansatz auf 4 kg Eis ausgetragen und der ausgefallene Feststoff abgesaugt. Das Produkt wird mit Wasser nachgewaschen und getrocknet.
Ausbeute: 310 g,
Schmelzbereich: 172-174°C

### Beispiel Z 5

### 2,4-Dichlor-5-fluor-3-N-hydroxyiminomethyl-benzoesäure

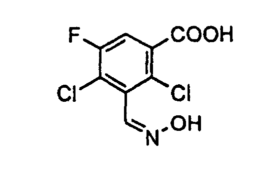

In einer Rührapparatur werden 80 g Hydroxylammoniumchlorid in 500 ml Ethanol vorgelegt und 200 ml 45 %-ige Natronlauge zugetropft und anschließend bei 40-45°C 200 g 2,4-Dichlor-5-fluor-3-formyl-benzoesäure eingetragen. Die Reaktion ist leicht exotherm und es wird 5 h bei 60°C nachgerührt. Nach Abkühlen auf Raumtemperatur wird durch Zutropfen von Salzsäure auf pH < 3 gestellt, das Produkt in tert.-Butyl-methyl-ether aufgenommen, die organische Phase abgetrennt und das Lösungsmittels abdestilliert. Als Rückstand erhält man 185 g 2,4-Dichlor-5-fluor-3-*N*-hydroxyiminomethyl-benzoesäure; Schmelzbereich: 190-194°C.

### Beispiel Z 6

### 2,4-Dichlor-3-cyano-5-fluor-benzoylchlorid

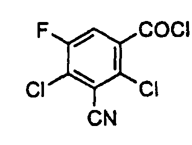

In einer Rührapparatur mit Dosiervorrichtung und Gasableitung über einen Rückflußkühler zu einem Wäscher werden 600 ml Thionylchlorid vorgelegt und bei 20°C 210 g 2,4-Dichlor-5-fluor-3-N-hydroxyiminomethyl-benzoesäure in dem Maße eingetragen, wie sich Chlorwasserstoff und Schwefeldioxid entwickelt. Nach der Zugabe wird die Mischung bis zum Ende der Gasentwicklung unter Rückfluß erhitzt. Anschließend wird destilliert, und im Siedebereich von 142-145°C/10 mbar werden 149 g 2,4-Dichlor-3-cyano-5-fluor-benzoylchlorid erhalten (Gehalt nach GC 98,1 %); Schmelzbereich: 73-75°C.

### Beispiel Z 7

### 3-Cyano-2,4,5-trifluor-benzoylfluorid

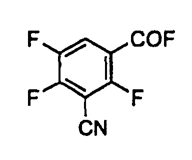

50 g Kaliumfluorid werden in 120 ml Tetramethylensulfon suspendiert und bei 15 mbar-zur Trocknung andestilliert (ca. 20 ml). Anschließend werden 50,4 g 2,4-Dichlor-3-cyano-5-fluor-benzoylchlorid zugefügt und unter Feuchtigkeitsausschluß für 12 Stunden bei 180°C Innentemperatur gerührt. Durch Vakuumdestillation werden 32,9 g 3-Cyano-2,4,5-trifluor-benzoylfluorid im Siedebereich von 98-100°C/12 mbar erhalten.

### Beispiel Z 8

### 3-Cyano-2,4,5-trifluor-benzoylchlorid

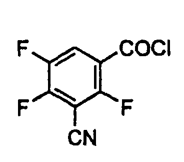

76,6 g 3-Cyano-2,4,5-trifluor-benzoylfluorid werden zusammen mit 1 g wasserfreiem Aluminiumchlorid bei 60-65°C vorgelegt und dann im Zuge der Gasentwicklung 25 g Siliciumtetrachlorid zugetropft. Nach Ende der Gasentwicklung bei 65°C wird im Vakuum destilliert. Im Siedebereich von 120-122°C/14 mbar gehen 73,2 g 3-Cyano-2,4,5-trifluor-benzoylchlorid über.

### Beispiel Z 9

### 1-(Toluol-4-sulfonyl)-pyrrolin

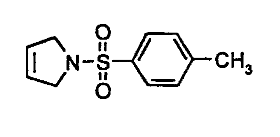

In einem 20 l HC4-Planschliffkessel werden 2,016 kg (17,6 mol) Methansulfonylchlorid in 121 Dichlormethan vorgelegt und bei -10°C Innentemperatur unter starker Kühlung (-34°C) eine Lösung von 705 g (8,0 mol) 2-Buten-1,4-diol in 1,944 kg (2,68 l, 19,2 mol) Triethylamin innerhalb von 30 Minuten zugetropft. Man erhält eine gelbe Suspension, die 1 Stunde bei -10°C nachgerührt und dann mit 4 l Wasser versetzt wird, wobei die Temperatur bis auf 0°C ansteigt. Die Suspension wird auf Raumtemperatur erwärmt, 10 Minuten bei Raumtemperatur gerührt und danach in eine 30 1-Scheidebirne eingezogen. Die Phasen werden getrennt (gute Phasentrennung) und die wäßrige Phase mit 2 l Dichlormethan ausgerührt. Die vereinigten Dichlormethanphasen werden in einem vorgekühlten 20 l HC4-Kessel vorgelegt und bei 0°C gehalten.

In einem weiteren 20 l HC4-Kessel mit Destillationsbrücke werden 1,37 kg (8,0 mol) Toluolsulfonsäureamid in 6 l Toluol vorgelegt. Man versetzt mit 3,2 kg 45%-iger Natronlauge, 0,81 Wasser und 130,5 g Tetrabutylammoniumhydrogensulfat, erhitzt auf maximal 40°C Innentemperatur und legt Vakuum an. Sodann wird die zuvor erhaltene Dichlormethanlösung (15,2 l) innerhalb von 1,5 Stunden zugetropft und dabei das Dichlormethan bei 450 mbar abdestilliert (Badtemperatur: 60°C). Während der Destillation erfolgt Schaumbildung. Am Ende liegt eine Lösung bei einer Innentemperatur von 33-40°C vor. Nach Beendigung der Zugabe wird weiter Dichlormethan abdestilliert, bis kaum noch Destillat übergeht (Dauer: ca. 85 Minuten; Innentemperatur 40°C bei 60°C Badtemperatur am Ende). Der Kesselinhalt wird warm in eine Scheidebirne überführt und der Kessel mit 5 l Wasser und 2 l Toluol bei 50°C nachgespült. Vor der Phasentrennung werden die Feststoffanteile in der Zwischenphase abgesaugt und mit 0,5 l Toluol nachgewaschen. Die organische Phase wird mit 2,4 l Wasser ausgerührt, abgetrennt und am Rotationsverdampfer bis zur Trockne eingedampft. Der feste Rückstand (1758 g) wird bei 50°C Badtemperatur in 1,6 l Methanol suspendiert, die Suspension in einen 10 l-Planschliffkolben überführt und der Kolben mit 2,4 l Diisopropylether nachgespült. Man erwärmt auf Rückflußtemperatur (59°C) und rührt noch 30 Minuten unter Rückfluß. Die Suspension wird auf 0°C abgekühlt, 1 Stunde bei 0°C gerührt, abgesaugt und mit 0,8 l einer kalten Mischung aus Methanol/Diisopropylether (1:1,5) gewaschen. Das Kristallisat wird unter einer Stickstoffatmosphäre bei 50°C/400 mbar getrocknet.

### Ausbeute: 1456 g (81,5 % der Theorie)

### Beispiel Z 10

### 3-(Toluol-4-sulfonyl)-6-oxa-3-aza-bicyclo[3.1.0]hexan

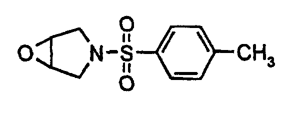

334,5 g (1,5 mol) 1-(Toluol-4-sulfonyl)-pyrrolin werden in 1,5 l Dichlormethan bei Raumtemperatur gelöst und innerhalb 15 Minuten mit einer Suspension von 408 g (ca. 1,65-1,77 mol) 70-75%-iger m-Chlorperbenzoesäure in 900 ml Dichlormethan (kühlt sich bei der Herstellung ab) versetzt. Man erhitzt 16 Stdn. unter Rückfluß (Test auf Peroxid mit KJ/Stärke-Papier zeigt noch Peroxidgehalt an), kühlt die Suspension auf 5°C, saugt die ausgefallene m-Chlorbenzoesäure ab und wäscht mit 300 ml Dichlormethan nach (Peroxidtest mit Niederschlag: negativ; Niederschlag verworfen). Das Filtrat wird zur Zerstörung überschüssigen Peroxids zweimal mit jeweils 300 ml 10 %-iger Natriumsulfit-Lösung gewaschen (Test auf Peroxid verläuft jetzt negativ), mit 300 ml gesättigter Natriumbicarbonat-Lösung extrahiert, mit Wasser gewaschen, mit Natriumsulfat getrocknet und auf ca. ein Viertel des Volumens eingeengt. Nochmals Test auf Peroxid: negativ. Die Mischung wird eingeengt und der feste Rückstand wird unter Eiskühlung mit 400 ml Isopropanol verrührt, der Niederschlag abgesaugt und bei 70°C im Vakuum getrocknet.
Ausbeute: 295 g (82,3 %),
Schmp.: 136-139°C,
DC (Dichlormethan/Methanol 98:2): 1 HK (Jodkammer)

### Beispiel Z 11

### trans-3-Hydroxy-4-(2-hydroxy-ethylamino)-1-(toluol-4-sulfonyl)-pyrrolidin

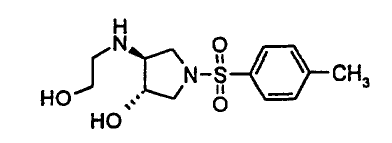

643,7 g (2,65 mol) 3-(Toluol-4-sulfonyl)-6-oxa-3-aza-bicyclo[3.1.0]hexan werden mit 318,5 ml Ethanolamin in 4 l Isopropanol 16 Stunden am Rückfluß gekocht. Nach DC-Kontrolle werden weitere 35,1 ml (insgesamt 5,86 mol) Ethanolamin zum Ansatz gegeben und erneut bis zum nächsten Morgen gekocht. Der Ansatz wird heiß abgesaugt und das Filtrat am Rotationsverdampfer auf 3,5 ltr eingeengt. Nach Animpfen und Rühren bei Raumtemperatur werden 3,5 l Diisopropylether dazugegeben und 6 Stunden bei 0° C nachgerührt. Das ausgefallene Kristallisat wird abgesaugt, mit 250 ml einer Mischung aus Isopropanol/Diisopropylether (1:1) und 2 mal mit je 300 ml Diisopropylether nachgewaschen und über Nacht im Hochvakuum getrocknet.
Ausbeute: 663,7 g (83 % der Theorie),
Gehalt: 96.1 % (Flächen-% nach HPLC).

### Beispiel Z 12

### trans-Toluol-4-sulfonsäure-{2-[[4-hydroxy-1-(toluol-4-sulfonyl)-pyrrolidin-3-yl]-(toluol-4-sulfonyl)-amino]-ethylester}

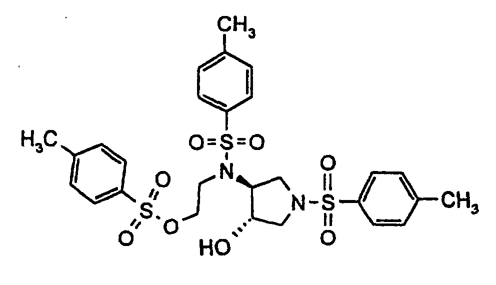

552 g (1,837 mol) trans-3-Hydroxy-4-(2-hydroxy-ethylamino)-1-(toluol-4-sulfonyl)-pyrrolidin werden unter Argon in 1,65 l Pyridin und 0,8 l Tetrahydrofuran gelöst, und bei -10° C werden portionsweise insgesamt 700 g (3,675 mol) p-Toluolsulfonylchlorid dazu gegeben. Der Ansatz wird anschließend 16 Stunden bei dieser Temperatur gerührt. Die Aufarbeitung erfolgt durch Zugabe von 4,3 l 18,5 %iger wäßriger Salzsäure, zweimaliger Extraktion mit Dichlormethan (3 l, 2 l), Waschen der vereinigten organischen Phasen mit gesättigter Natriumhydrogencarbonatlösung (3 l, 2 l), Trocknen über Natriumsulfat, Absaugen und Abdestillieren des Lösungsmittels im Vakuum. Der Rückstand wird über Nacht an der Ölpumpe getrocknet und roh in die nächste Reaktion eingesetzt. Es ergaben sich 1093 g als harter Schaum (Gehalt [Flächen-% nach HPLC]: 80 % Tris-Tosyl-Produkt und 13 % Tetra-Tosyl-Produkt; Ausbeute siehe nächste Stufe).

### Beispiel Z 13

### rac. trans-5,8-Bis-tosyl-2-oxa-5,6-diazabicyclo[4.3.0]nonan

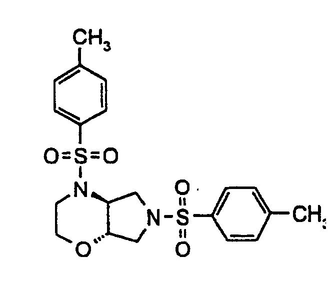

1092 g roher trans-Toluol-4-sulfonsäure-{2-[[4-hydroxy-1-(toluol-4-sulfonyl)-pyrrolidin-3-yl]-(toluol-4-sulfonyl)-amino]-ethylester} werden in 9,4 l Tetrahydrofuran gelöst und bei 0-3° C mit 1,4 l einer 1,43 molaren Lösung von Natriumhydroxid in Methanol umgesetzt Nach einer halben Stunde bei dieser Temperatur wurden 2,1 l Wasser und 430 ml verdünnte (2:1) Essigsäure zum Ansatz gegeben und mit zuvor isolierten Kristallen von trans-Toluol-4-sulfonsäure-{2-[[4-hydroxy-1-(toluol-4-sulfonyl)-pyrrolidin-3-yl]-(toluol-4-sulfonyl)-amino]-ethylester} angeimpft. Die Suspension wird über Nacht bei 0 bis -4° C gerührt. Am nächsten Morgen werden die Kristalle abgesaugt, zweimal mit je 400 ml kaltem Gemisch aus Tetrahydrofuran/Wasser (4:1) gewaschen und bei 3 mbar bei 50° C über Nacht getrocknet.
Ausbeute: 503 g weiße Kristalle (62,7 % der Theorie über 2 Stufen),
Gehalt: 99,7 % (Flächen-% nach HPLC).

### Beispiel Z 14

### Präparative chromatographische Racemattrennung von rac. trans-5,8-Bis-tosyl-2-oxa-5,6-diazabicyclo[4.3.0]nonan

Die Chromatographie des Racemats erfolgt bei Raumtemperatur in einer Säule (Innendurchmesser 75mm), welche mit 870 g einer chiralen stationären Phase (kieselgelgebundenes Poly(N-methacryloyl-L-leucin-d-menthylamid) auf Basis des mercaptomodifizierten Kieselgels Polygosil 100, 10 µm; siehe EP-A-0 379 917) gefüllt ist (Betthöhe: ca. 38 cm). Die Detektion erfolgt mit Hilfe eines UV-Detektors bei 254 nm. Für die Probenaufgabe verwendet man eine Lösung einer Konzentration von 100 g rac. trans-5,8-Bis-tosyl-2-oxa-5,6-diazabicyclo[4.3.0]nonan in 3000 ml. Tetrahydrofuran. Ein Trenncyclus wird unter folgenden Bedingungen durchgeführt: Mit Hilfe einer Pumpe wird während 2 min bei einem Fluß von 50 ml/min ein Teil der Probenlösung und gleichzeitig bei einem Fluß von 50 ml/min reines n-Heptan auf die Säule gefördert. Danach wird 18 min lang mit einem Gemisch aus n-Heptan/Tetrahydrofuran (3/2 vol/vol) bei einem Fluß von 100 ml/min eluiert. Anschließend folgt während 3 min bei einem Fluß von 100 ml/min eine Elution mit reinem Tetrahydrofuran. Danach wird weiter mit n-Heptan/Tetrahydro-furan (3/2 vol/vol) eluiert. Dieser Cyclus wird mehrfach wiederholt.

Das zuerst eluierte Enantiomer ist das (1R,6R)-5,8-Bis-tosyl-2-oxa-5,6-diazabicyclo-[4.3.0]nonan, das durch Einengen isoliert wird. Das Eluat des stärker retardierenden Enantiomeren wird im Vakuum weitgehend eingedampft, das ausgefallene Kristallisat abgesaugt und getrocknet. Aus der Trennung von 179 g Racemat werden auf diese Weise 86,1 g (96,2 % der Theorie) des Enantiomeren (1S,6S)-5,8-Bis-tosyl-2-oxa-5,6-diazabicyclo[4.3.0]nonan mit einer Reinheit von > 99 % ee isoliert.

### Beispiel Z 15

### (1R,6R)-2-Oxa-5,6-diazabicyclo[4.3.0]nonan-Dihydrobromid

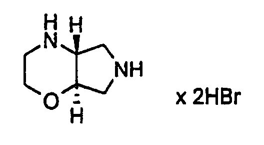

38,3 g (87 mmol) (1R,6R)-5,8-Bis-tosyl-2-oxa-5,6-diazabicyclo[4.3.0]nonan in 500 ml 33-%iger HBr/Eisessig mit 10 g Anisol versetzt und 4 Stdn. auf 60°C (Bad) erwärmt. Nach Stehen über Nacht wird die Suspension gekühlt, der Niederschlag abgesaugt, mit 100 ml absol. Ethanol gewaschen und bei 70°C im Hochvakuum getrocknet.
Ausbeute: 23,5 g (93 %) weißes Festprodukt,
Schmp.: 309-310°C (Zers.),
DC (Dichlormethan/Methanol/17%-wäßr. Ammoniak 30:8:1): 1 HK,
[α]_{D}: + 0,6° (c = 0,53, H₂O) (schwankend).

### Beispiel Z 16

### (1S,6S)-2-Oxa-5,6-diazabicyclo[4.3.0]nonan-Dihydrobromid

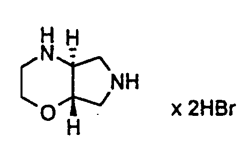

Analog Beispiel Z 15 wird aus (1S,6S)-5,8-Bis-tosyl-2-oxa-5,6-diazabicyclo[4.3.0]nonan (1S,6S)-2-Oxa-5,6-diazabicyclo[4.3.0]nonan-Dihydrobromid erhalten.

### Beispiel Z 17

### (1R,6R)-2-Oxa-5,8-diazabicyclo[4.3.0]nonan

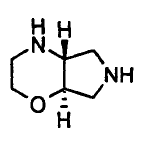

1. Methode: 5,8 g (20 mmol) (lR,6R)-2-Oxa-5,8-diazabicyclo[4.3.0]nonan-Dihydrobromid werden in 100 ml Isopropanol bei Raumtemperatur suspendiert, mit 2,4 g (42,9 mmol) pulverisiertem Kaliumhydröxid versetzt und während circa 1 Stunde im Ultraschallbad belassen. Die Suspension wird im Eisbad gekühlt, filtriert, das ungelöste Salz mit Isopropanol gewaschen und das Filtrat eingeengt und im Kugelrohrofen bei 150-230°C Ofentemperatur und 0,7 mbar destilliert. Man erhält 2,25 g (87,9 % der Theorie) eines zähen Öles, das durchkristallisiert. [α]_{D}: -21,3° (c = 0,92, CHCl₃) Entsprechend läßt sich diese Umsetzung auch in Ethanol durchführen.
2. Methode: Eine homogeniesierte Mischung aus (1R,6R)-2-Oxa-5,8-diazabicyclo-[4.3.0]nonan-Dihydrobromid und 620 mg (11 mmol) pulverisiertem Kaliumhydroxid wird trocken in einer Kugelrohrapparatur bei 0,2 mbar und steigender Ofentemperatur bis 250°C destilliert. Man erhält 490 mg (76,6 % der Theorie) (1R,6R)-2-Oxa-5,8-diazabicyclo[4.3.0]nonan als zähes Öl, das langsam kristallisiert.
3. Methode: 100 g feuchter, vorbehandelter Kationenaustauscher (Dowex 50WX, H⁺-Form, 100-200 mesh, Kapazität: 5,1 meq/g trocken oder 1,7 meq/ml) werden in eine Säule gefüllt, mit ca. 200 ml 1 n HCl aktiviert und mit 3 l Wasser neutral gewaschen. Eine Lösung von 2,9 g (10 mmol) (1R,6R)-2-Oxa-5,8-diazabicyclo[4.3.0]nonan-Dihydrobromid in 15 ml Wasser wird auf den Ionenaustauscher gegeben und anschließend mit 2 l Wasser gewaschen und mit 1 l ca. 1 n-Ammoniaklösung eluiert. Das Eluat wird i. Vak. eingeengt.
   Ausbeute: 1,3 g zähes Öl (quant.),
   DC (Dichlormethan/Methanol/17%-NH₃ 30:8:1): 1 HK,
   GC: 99,6 % (Fläche).

### Beispiel Z 18

### (1S,6S)-2-Oxa-5,8-diazabicyclo[4.3.0]nonan

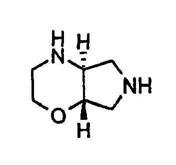

Analog Beispiel Z 17 wird aus (1S,6S)-2-Oxa-5,8-diazabicyclo[4.3.0]nonan-Dihydrobromid die freie Base (1S,6S)-2-Oxa-5,8-diazabicyclo[4.3.0]nonan hergestellt.

### Beispiel Z 19

### 2-(2,4-Dichlor-3-cyano-5-fluor-benzoyl)-3-dimethylamino-acrylsäureethylester

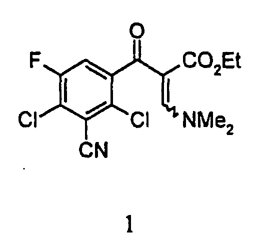

Zu einer Lösung von 626 g (4,372 mol) 3-Dimethylamino-acrylsäureethylester und 591 g (4,572 mol) Ethyl-diisopropyl-amin (Hünig-Base) in 1060 ml Dichlormethan wird bei Raumtemperatur beginnend eine Lösung von 1075 g 2,4-Dichlor-3-cyano-5-fluorbenzoylchlorid (ca. 94%ig, entsprechend 1010,5 g = 4,00 mol) in 850 ml Dichlormethan getropft. Dabei steigt die Temperatur auf ca. 50-55°C (Zutropfdauer ca. 90 Minuten). Anschließend wird 2 Stunden bei 50°C nachgerührt und das Reaktionsgemisch dann ohne weitere Aufarbeitung in die nächste Stufe eingesetzt.

### Beispiel Z 20

### 2-(2,4-Dichlor-3-cyano-5-fluor-benzoyl)-3-cyclopropylamino-acrylsäureethylester

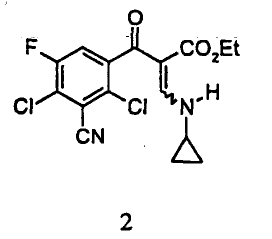

Zu dem Reaktionsgemisch aus der obigen Stufe werden unter Kühlung bei ca. 15°C 306 g (5,1 mol) Eisessig getropft. Dann werden unter weiterer Kühlung bei ca. 10-15°C 267,3 g (4,68 mol) Cyclopropylamin zugetropft. Sofort danach wird das Reaktionsgemisch unter Eiskühlung mit 1300 ml Wasser versetzt und 15 Minuten gut verrührt. Die Dichlormethan-Phase wird abgetrennt und in die nächste Stufe eingesetzt.

### Beispiel Z 21

### 7-Chlor-8-cyano-1-cyclopropyl-6-fluor-1,4-dihydro-4-oxo-3-chinolincarbonsäureethylester

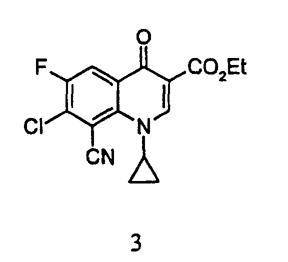

Zu einer auf 60-70°C geheizten. Suspension von 353 g (2,554 mol) Kaliumcarbonat in 850 ml N-Methylpyrrolidon wird die Dichlormethan-Phase aus der Vorstufe getropft (ca. 90 Minuten). Während der Zugabe wird gleichzeitig Dichlormethan aus dem Reaktionsgemisch abdestilliert. Anschließend wird das Reaktionsgemisch noch 5 ½ Stunden bei 60-70°C gut gerührt. Man läßt auf ca. 50°C abkühlen und destilliert bei einem Vakuum von ca. 250 mbar restliches Dichlormethan ab. Bei Raumtemperatur werden dann unter Eiskühlung 107 ml 30%ige Salzsäure zugetropft, wodurch ein pH-Wert von 5-6 eingestellt wird. Dann werden unter Eiskühlung 2200 ml Wasser zugegeben. Das Reaktionsgemisch wird 15 Minuten gut gerührt, der Feststoff dann abgesaugt und auf der Nutsche zweimal mit je 1000 ml Wasser und dreimal mit je 1000 ml Ethanol gewaschen und anschließend im Vakuumtrockenschrank bei 60°C getrocknet.
Ausbeute: 1200 g (89,6% der Theorie).

Dieses Produkt kann gegebenenfalls noch gereinigt werden, indem der Feststoff in 2000 ml Ethanol 30 Minuten bei Rückfluß verrührt wird. Man saugt heiß ab, wäscht mit 500 ml Ethanol nach und trocknet bei 60°C im Vakuum.
Schmelzpunkt: 180-182°C.
¹H-NMR(400 MHz, CDCl₃): d = 1,2-1,27 (m; 2H), 1,41 (t; 3H), 1,5-1,56 (m; 2H), 4,1-4,8 (m; 1H), 4,40 (q; 2H), 8,44 (d, J=8,2 Hz; H), 8,64 (s; 1H) ppm.

### Beispiel Z 22

### 7-Chlor-8-cyano-1-cyclopropyl-6-fluor-1,4-dihydro-4-oxo-3-chinolincarbonsäure

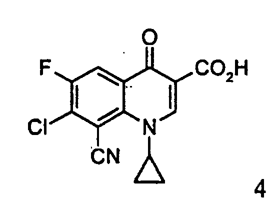

33,8 g (0,1 mol) 7-Chlor-8-cyano-1-cyclopropyl-6-fluor-1,4-dihydro-4-oxo-3-chinolincarbonsäureethylester werden in einer Mischung aus 100 ml Essigsäure, 20 ml Wasser und 10 ml konzentrierter Schwefelsäure 3 Stunden unter Rückfluß erhitzt Nach dem Abkühlen wird auf 100 ml Eiswasser ausgegossen, der ausgefallene Niederschlag abgesaugt, mit Wasser'und Ethanol gewaschen und bei 60°C im Vakuum getrocknet.
Ausbeute: 29,6 g (96 % der Theorie),
Schmelzpunkt: 276-277°C (unter Zersetzung)

### Herstellung der Wirkstoffe

### Beispiel 1

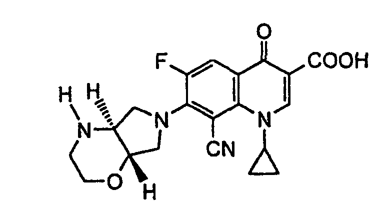

### A) 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure

1,00 g (3,26 mmol) 7-Chlor-8-cyano-1-cyclopropyl-6-fluor-1,4-dihydro-4-oxo-3-chinolincarbonsäure werden mit 501 mg (3,91 mmol) (1S,6S)-2-Oxa-5,8-diazabicyclo[4.3.0]nonan und 0,9 ml Triethylamin in 30 ml Acetonitril 25 Stunden unter Argon bei 40-45°C gerührt. Alle flüchtigen Komponenten werden i. Vak. entfernt und der Rückstand aus Ethanol umkristallisiert.
Ausbeute: 1,22 g (94 %)
Schmelzpunkt: 294°C (unter Zersetzung)

### B) 8-Cyano-1-cyclopropyl-6-fluor-7-((1S,6S)-2-oxa-5,8-diazabicyclo[4.3.0]nonan-8-yl)-1,4-dihydro-4-oxo-3-chinolincarbonsäure-Hydrochlorid

200 mg (0,63 mmol) 8-Cyano-1-cyclopropyl-6,7-difluor-1,4-dihydro-4-oxo-3-chinolincarbon-säureethylester werden mit 97 mg (0,75 mmol) (1S,6S)-2-Oxa-5,8-diazabicyclo[4.3.0]nonan und 0,17 ml Triethylamin in 3 ml Acetonitril 2 Stunden unter Argon bei 40-45°C gerührt. Alle flüchtigen Komponenten werden i. Vak. entfernt, der Rückstand mit Wasser versetzt, Unlösliches abfiltriert und das Filtrat mit Dichlormethan extrahiert. Man trocknet die organische Phase über Natriumsulfat und engt anschließend i. Vak. ein. Der resultierende Rückstand wird in 6 ml Tetrahydrofuran und 2 ml Wasser gelöst und mit 30 mg (0,72 mmol) Lithiumhydroxid-Monohydrat versetzt. Nach 16 Stunden Rühren bei Raumtemperatur wird mit verd. Salzsäure angesäuert und der resultierende Niederschlag abgesaugt und getrocknet.
Ausbeute: 155 mg (57 %)
Schmelzpunkt: > 300°C

### C) 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure-Hydrochlorid

1 g (2,5 mmol) 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure wird in 20 ml Wasser suspendiert, die Suspension mit 10 ml In-Salzsäure versetzt und 3 Stunden bei Raumtemperatur verrührt. Der erhaltene Niederschlag wird abgesaugt, mit Ethanol gewaschen und bei 80°C im Hochvakuum getrocknet.
Ausbeute: 987 mg (90,6 % der Theorie),
Schmelzpunkt: 314-316°C (unter Zersetzung).

### D) 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure-Hydrochlorid

86,4 g (217 mmol) 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure werden bei Raumtemperatur in 963 ml Wasser und 239 ml 1 n wäßriger Natronlauge gelöst. Nach Filtration und Nachwaschen mit 200 ml Wasser wird mit 477 ml In wäßriger Salzsäure versetzt und das ausgefallene Kristallisat bei 95°C bis 100°C in Lösung gebracht. Die Lösung wird über Nacht abgekühlt, das ausgefallene Kristallisat abgesaugt und dreimal mit je 500 ml Wasser gewaschen und im Vakuum getrocknet.
Ausbeute 90 g (94,7 % der Theorie),
Gehalt: > 99 % (Flächen-% nach HPLC); 99,6 % ee.
[α]_{D}²³:-112° (c = 0,29, 1N NaOH).

### Beispiel 2

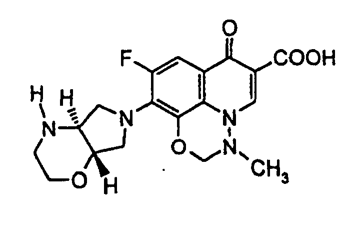

### 9-Fluor-3-methyl-10-((1S,6S)-2-oxa-5,8-diazabicyclo[4.3.0]non-8-yl)-7-oxo-2,3-dihydro-7H-pyrido[1,2,3-d,e][1,3,4]benzoxadiazin-6-carbonsäure

100 mg (0,354 mmol) 9,10-Difluor-3-methyl-7-oxo-2,3-dihydro-7H-pyrido[1,2,3-d,e]-[1,3,4]benzoxadiazin-6-carbonsäure werden mit 91 mg (0,71 mmol) (1S,6S)-2-Oxa-5,8-diazabicyclo[4.3.0]nonan in 3 ml DMSO eine Stunde unter Argon auf 120°C erwärmt. Die Mischung wird im Hochvakuum eingeengt, der Rückstand aus Ethanol umkristallisiert und getrocknet.
Ausbeute: 106 mg (77 % der Theorie)
Schmelzpunkt: 205°C (unter Zersetzung)

### Beispiel 3

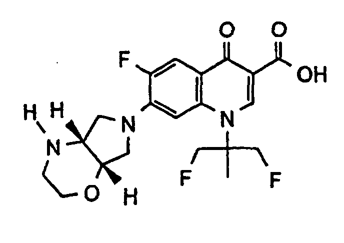

### 1-(1-Fluormethyl-1-methyl-2-fluorethyl)-6-fluor-7-[(1S,6R)-2-oxa-5,8-diaza-bicyclo[4.3.0]non-8-yl]-1,4-dihydro-4-oxo-3-chinolincarbonsäure

Eine Lösung aus 1-(1-Fluormethyl-1-methyl-2-fluorethyl)-6,7-difluor-1,4-dihydro-4-oxo-3-chinolincarbonsäure (400 mg, 1,26 mmol), (1S,6R)-2-Oxa-5,8-diazabicyclo[4.3.0]nonan (176 mg, 1,39 mmol) und 1,4-Diazabicyclo[2.2.2]octan (141 mg, 1,26 mmol) in absol. Acetonitril (20 ml) wird über Nacht unter Rückfluß erhitzt. Nach dem Abkühlen des Reaktionsgemisches auf Raumtemperatur werden die ausgefallenen Kristalle abfiltriert und mit Acetonitril gewaschen.
Ausbeute: 392 mg (73% d. Th.)
Schmelzpunkt: 245°C

### Beispiel 4

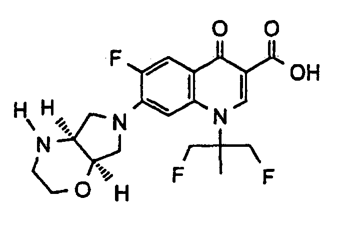

### 1-(1-Fluormethyl-1-methyl-2-fluorethyl)-6-fluor-7-[(1R,6S)-2-oxa-5,8-diaza-bicyclo[4.3.0]non-8-yl]-1,4-dihydro-4-oxo-3-chinolincarbonsäure

Die Titelverbindung wird analog zur Vorschrift des Beispiels 3 durch Umsetzung mit (1R,6S)-2-Oxa-5,8-diazabicyclo[4.3.0]nonan hergestellt.
Ausbeute: 58% d. Th.
Schmelzpunkt: >250°C

### Beispiel 5

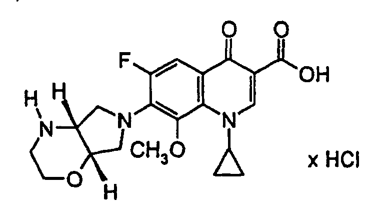

### 1-(Cyclopropyl)-6-fluor-8-methoxy-7-[(1S,6R)-2-oxa-5,8-diazabicyclo[4.3.0]non-8-yl]-1,4-dihydro-4-oxo-3-chinolincarbonsäure-Hydrochlorid

Die Titelverbindung wird analog zur Vorschrift des Beispiels 3 durch Umsetzung mit (1S,6R)-2-Oxa-5,8-diazabicyclo[4.3.0]nonan hergestellt. Das Rohprodukt wird säulen-chromatographisch gereinigt (CH₂Cl₂/MeOH/AcOH, 10 : 5 : 0,5), wobei das Produkt als Acetat-Salz anfällt. Nach Zugabe von Methanol und 1N HCl und Einengen der Lösung im Vakuum wird das Hydrochlorid kristallin erhalten.
Ausbeute: 67% d. Th.
Schmelzpunkt: >250°C

### Beispiel 6

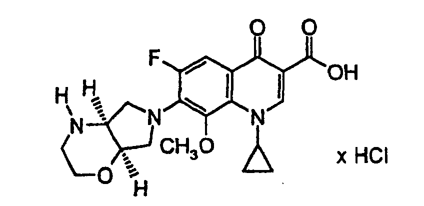

### 1-Cyclopropyl-6-fluor-8-methoxv-7-[(1R,6S)-2-oxa-5.8-diazabicyclo[4.3.0]non-8-yl]-1,4-dihydro-4-oxo-3-chinolincarbonsäure-Hydrochlorid

Die Titelverbindung wird analog zur Vorschrift des Beispiels 5 durch Umsetzung mit (1R,6S)-2-Oxa-5,8-diazabicyclo[4.3.0]nonan hergestellt.
Ausbeute: 37% d. Th.
Schmelzpunkt: >250°C

### Beispiel 7

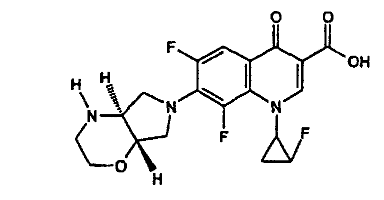

### 1-(cis-2-Fluorcyclopropyl)-6,8-difluor-1,4-dihydro-7-(1S,6S-2-oxa-5,8-diazabicyclo[4.3.0]non-8-yl)-4-oxo-3-chinolincarbonsäure

Eine Mischung von 3,6 g (12 mmol) 1-(cis-2-Fluorcyclopropyl)-6,7,8-trifluor-1,4-dihydro-4-oxo-3-chinolincarbonsäure in 50 ml ml Acetonitril und 25 ml Dimethylformamid mit 3,36 g (30 mmol) 1,4-Diazabicyclo[2.2.2]octan und 3,7 g (12,8 mmol) 1S,6S-2-Oxa-5,8-diazabicyclo[4.3.0]nonan-Dihydrobromid wird 1 Stunde unter Rückfluß erhitzt. Die Mischung wird eingeengt, der Rückstand mit etwas Wasser versetzt und 30 Minuten im Ultraschallbad behandelt. Der ungelöste Niederschlag wird abgesaugt, mit Wasser gewaschen und bei 80°C im Hochvakuum getrocknet.
Ausbeute: 4,2 g (86 % der Theorie)
Schmelzpunkt: 274-276°C (unter Zersetzung).

### Beispiel 8

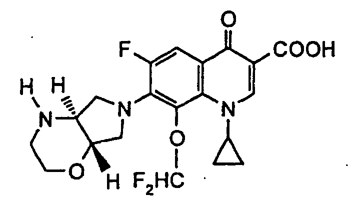

### 1-Cyclopropyl-8-difluormethoxy-6-fluor-1,4-dihydro-7-((1S,6S)-2-oxa-5,8-diazabicyclo[4.3.0]non-8-yl)-4-oxo-3-chinolincarbonsäure

Eine Mischung von 166 mg (0,5 mmol) 1-Cyclopropyl-8-difluormethoxy-6,7-difluor-1,4-dihydro-4-oxo-3-chinolincarbonsäure in 1,5 ml ml Acetonitril und 0,75 ml Dimethylformamid mit 73 mg (0,65 mmol) 1,4-Diazabicyclo[2.2.2]octan und 100 mg (0,78 mmol) 1S,6S-2-Oxa-5,8-diazabicyclo[4.3.0]nonan wird 1 Stunde unter Rückfluß erhitzt. Die Mischung wird eingeengt, der Rückstand mit etwas Wasser versetzt und 20 Minuten im Ultraschallbad behandelt. Der ungelöste Niederschlag wird abgesaugt, mit Wasser gewaschen und bei 80°C im Hochvakuum getrocknet.
Ausbeute: 164 mg (75 % der Theorie)
Schmelzpunkt: 209-211°C (unter Zersetzung).
[α]_{D}²⁵: -250° (c = 0,25, DMF).

### Beispiel 9

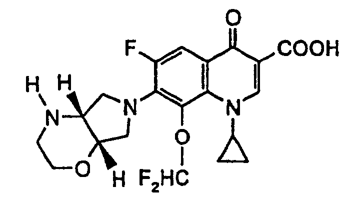

Analog Beispiel 8 wird 1-Cyclopropyl-8-difluormethoxy-6-fluor-1,4-dihydro-7-((1S,6R)-2-oxa-5,8-diazabicyclo[4.3.0]non-8-yl)-4-oxo-3-chinolincarbonsäure vom Schmelzpunkt: 181-182°C (unter Zersetzung) erhalten.
[α]_{D}²⁵EQ: -23° (c = 0,25, DMF).

### Beispiel 10

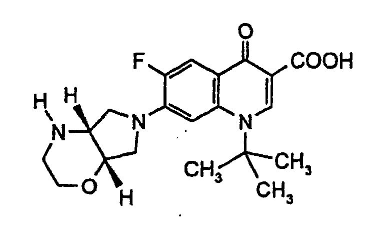

Analog Beispiel 8 wird 1-tert.-Butyl-6-fluor-1,4-dihydro-7-((1S,6R)-2-oxa-5,8-diazabicyclo[4.3.0]non-8-yl)-4-oxo-3-chinolincarbonsäure vom Schmelzpunkt: 224-226°C (unter Zersetzung) erhalten.
[α]_{D}²⁵: +70° (C = 0.25, DMF)

### Beispiel 11

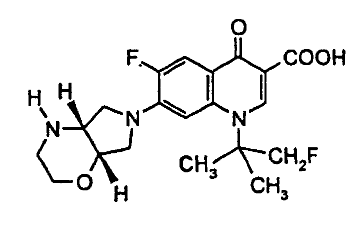

Analog Beispiel 8 wird 6-Fluor-1-(fluor-tert.-butyl)-1,4-dihydro-7-((1S,6R)-2-oxa-5,8-diazabicyclo[4.3.0]non-8-yl)-4-oxo-3-chinolincarbonsäure vom
Schmelzpunkt: 243-244°C (unter Zersetzung) erhalten.
[α]_{D}²⁵: + 71° (c = 0.25, DMF)

### Beispiel 12

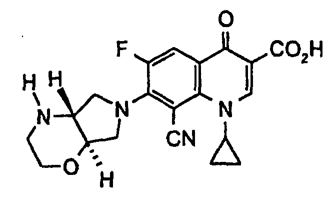

### A) 8-Cyano-1-cyclopropyl-6-fluor-7-((1R,6R)-2-oxa-5,8-diazabicyclo[4.3.0]non-8-yl)-1,4-dihydro-4-oxo-3-chinolincarbonsäure

1. Methode: 310 mg (1 mmol) 7-Chlor-8-cyano-1-cyclopropyl-6-fluor-1,4-dihydro-4-oxo-3-chinolincarbonsäure werden mit 300 mg (1,05 mmol) (1R,6R)-2-Oxa-5,8-diazabicyclo[4.3.0]nonan-Dihydrobromid und 610 mg (6 mmol) Triethylamin in einer Mischung aus 4 ml Acetonitril und 2 ml DMF 1 Stunde unter Rückfluß erhitzt. Nach DC und HPLC ist keine 7-Chlor-8-cyano-1-cyclopropyl-6-fluor-1,4-dihydro-4-oxo-3-chinolincarbonsäure mehr nachweisbar. Die Mischung wird über Nacht zur Kristallisation in den Kühlschrank gestellt, der Niederschlag abgesaugt, mit Wasser gewaschen und bei 80°C im Hochvakuum getrocknet.
Ausbeute: 335 mg (84 %),
Schmelzpunkt: 295-296°C (unter Zersetzung)

2. Methode: 920 mg (3 mmol) 7-Chlor-8-cyano-1-cyclopropyl-6-fluor-1,4-dihydro-4-oxo-3-chinolincarbonsäure werden mit 480 mg (3,75 mmol) (1R,6R)-2-Oxa-5,8-diazabicyclo[4.3.0]nonan und 0,9 ml Triethylamin in 25 ml Acetonitril unter Stickstoff 4 Stunden bei 45°C gerührt; Zugabe von weiteren 0,5 ml Triethylamin, dann noch 16 Stunden bei 60°C gerührt. Die Suspension wird im Eisbad gekühlt, der Niederschlag abgesaugt, mit Ethanol gewaschen und bei 70°C im Vakuum getrocknet.
Ausbeute: 1,05 g (88 %),
Schmelzpunkt: 294°C (unter Zersetzung),
[α]_{D}: +103,6° (c = 0,33; 1N NaOH),
HPLC: 99,9 % (Fläche).

### B) 8-Cyano-1-cyclopropyl-6-fluor-7-((1R,6R)-2-oxa-5,8-diazabicyclo[4.3.0]non-8-yl)-1,4-dihydro-4-oxo-3-chinolincarbonsäure-Hydrochlorid

Analog Beispiel 1C wird 8-Cyano-1-cyclopropyl-6-fluor-7-((lR,6R)-2-oxa-5,8-diazabicyclo[4.3.0]non-8-yl)-1,4-dihydro-4-oxo-3-chinolincarbonsäure mit Salzsäure umgesetzt.

### Beispiel 13

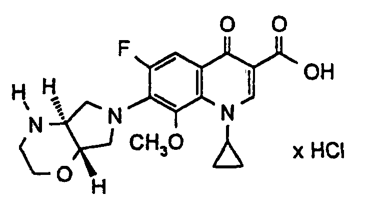

### 1-Cyclopropyl-6-fluor-8-methoxy-7-((1S,6S)-2-oxa-5,8-diazabicyclo[4.3.0]nonan-8-yl-1,4-dihydro-4-oxo-3-chinolincarbonsäure Hydrochlorid

Die Titelverbindung wird analog zur Vorschrift des Beispiels 5 durch Umsetzung mit (1S,6S)-2-Oxa-5,8-diazabicyclo[4.3.0]nonan hergestellt. Das Rohprodukt wird säulenchromatographisch gereinigt (CH₂Cl₂/MeOH/AcOH, 10 : 5 : 0,5), wobei das Produkt als Acetat-Salz anfällt. Nach Zugabe von Methanol und 1N HCl und Einengen der Lösung im Vakuum wird das Hydrochlorid kristallin erhalten.
Schmelzpunkt: >250°C

### Beispiel 14

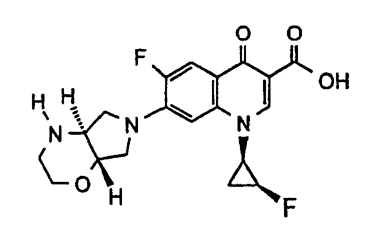

### 6-Fluor-1-((1R,2S)-2-fluorcyclopropyl)-7-((1S,6S)-2-oxa-5,8-diazabicyclo[4.3.0]nonan-8-yl)-1,4-dihydro-4-oxo-3-chinolincarbonsäure

Die Titelverbindung wird analog zur Vorschrift des Beispiel 8 durch Umsetzung von 6,7-Difluor-1-((1R,2S)-2-fluorcyclopropyl)-1,4-dihydro-4-oxo-3-chinolincarbonsäure mit (1S,6S)-2-Oxa-5,8-diazabicyclo[4.3.0]nonan dargestellt.
Schmelzpunkt: >250°C

### Beispiele 15-21

Analog Beispiel 8 werden unter Verwendung von (1R,6S)-2-oxa-5,8-diazabicyclo-[4.3.0]nonan die folgenden Verbindungen erhalten, die teilweise durch Lösen in halbkonzentrierter Salzsäure, Eindampfen und Behandeln mit Ethanol als Hydrochloride isoliert wurden:

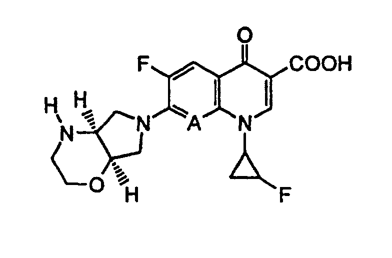

### Beispiel 15

6-Fluor-1-(cis-2-fluorcyclopropyl)-1,4-dihydro-7-((1R,6S)-2-oxa-5,8-diazabicyclo[4.3.0]non-8-yl)-4-oxo-3-chinolincarbonsäure (A = CH), Schmelzpunkt: 236-238°C (unter Zersetzung);

### Beispiel 16

6,8-Difluor-1-(cis-2-fluorcyclopropyl)-1,4-dihydro-7-((1R,6S)-2-oxa-5,8-diazabicyclo[4.3.0]non-8-yl)-4-oxo-3-chinolincarbonsäure-Hydrochlorid (A = CF; x HCl), Schmelzpunkt: 275-280°C (unter Zersetzung);

### Beispiel 17

8-Chlor-6-fluor-1-(cis-2-fluorcyclopropyl)-1,4-dihydro-7-((1R,6S)-2-oxa-5,8-diazabicyclo[4.3.0]non-8-yl)-4-oxo-3-chinolincarbonsäure-Hydrochlorid (A = CCl; x HCl), Schmelzpunkt: 210-215°C (unter Zersetzung);

### Beispiel 18

6-Fluor-1-(cis-2-fluoryclopropyl)-1,4-dihydro-7-((1R,6S)-2-oxa-5,8-diazabicyclo[4.3.0]non-8-yl)-4-oxo-1,8-naphthyridin-3-carbonsäure-Hydrochlorid (A = N; x HCl),
Schmelzpunkt: 281-284°C (unter Zersetzung);

### Beispiel 19

6-Fluor-1-(trans-2-fluorcyclopropyl)-1,4-dihydro-7-((1R,6S)-2-oxa-5,8-diazabicyclo[4.3.0]non-8-yl)-4-oxo-3-chinolincarbonsäure (A = CH), Schmelzpunkt: 270-274°C (unter Zersetzung);

### Beispiel 20

8-Chlor-6-fluor-1-(trans-2-fluorcyclopropyl)-1,4-dihydro-7-((1R,6S)-2-oxa-5,8-diazabicyclo[4.3.0]non-8-yl)-4-oxo-3-chinolincarbonsäure (A = CCl), Schmelzpunkt: 160-164°C (unter Zersetzung);

### Beispiel 21

6-Fluor-1-(trans-2-fluorcyclopropyl)-1,4-dihydro-7-((1R,6S)-2-oxa-5,8-diazabicyclo[4.3.0]non-8-yl)-4-oxo-1,8-naphthyridin-3-carbonsäure (A = N), Schmelzpunkt: 310-314°C (unter Zersetzung).

## Patentansprüche

1. Verwendung von Verbindungen der allgemeinen Formel (I) 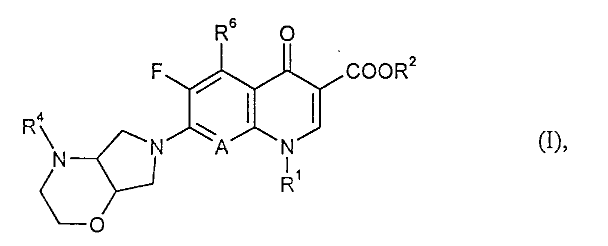 in welcher
R¹ für gegebenenfalls durch Halogen ein- oder zweifach substituiertes Alkyl mit 1 bis 4 C-Atomen, gegebenenfalls durch 1 oder 2 Fluoratome substituiertes Phenyl oder gegebenenfalls durch 1 oder 2 Fluoratome substituiertes Cyclopropyl steht,
R² für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
A für N oder C-R³ steht, wobei
R³ für Wasserstoff, Halogen, Methyl, Methoxy, Difluormethoxy oder Cyano steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verbunden ist, bilden kann,
R⁴ für Wasserstoff, Benzyl, C₁-C₃-Alkyl, (5-Methyl-2-oxo-1,3-dioxol-4-yl)-methyl, Reste der Strukturen -CH=CH-COOR⁵, -CH₂CH₂COOR⁵, -CH₂CH₂CN, -CH₂CH₂COCH₃, -CH₂COCH₃, in denen
R⁵ für Methyl oder Ethyl,
R⁶ für Wasserstoff, Amino, Hydroxy, Methyl oder Halogen steht,
in Form von Racematen, Diastereomerengemischen oder als enantiomerenreine oder diastereomerenreine Verbindungen von deren pharmazeutisch verwendbaren Hydraten und/oder Salzen zur Herstellung von Arzneimitteln zur Therapie von Helicobacter-pylori-Infektionen und den damit assoziierten gastroduodenalen Erkrankungen.

2. Verwendung von Verbindungen der Formel (I), in welcher
R¹ für gegebenenfalls durch Fluor ein- oder zweifach substituiertes tert.-Butyl oder gegebenenfalls durch 1 Fluoratom substituiertes Cyclopropyl steht,
R² für Wasserstoff, Alkyl mit 1 bis 4 Kohlenstoffatomen oder (5-Methyl-2-oxo-1,3-dioxol-4-yl)-methyl steht,
A für C-R³ steht, wobei
R³ für Wasserstoff, Fluor, Methoxy, Difluormethoxy oder Cyano steht oder auch gemeinsam mit R¹ eine Brücke der Struktur -*O-CH₂-CH-CH₃ oder -*O-CH₂-N-CH₃, wobei das mit * markierte Atom mit dem Kohlenstoffatom von A verbunden ist, bilden kann,
R⁴ für Wasserstoff, C₁-C₃-Alkyl, Reste der Strukturen -CH₂CH₂COOR⁵, -CH₂CH₂CN, -CH₂COCH₃ steht, in denen
R⁵ für Methyl oder Ethyl steht,
R⁶ für Wasserstoff, Amino oder Methyl steht,
von deren pharmazeutisch verwendbaren Hydraten und/oder Salzen zur Herstellung von Arzneimitteln zur Therapie von Helicobacter-pylori-Infektionen und den damit assoziierten gastroduodenalen Erkrankungen.

3. Verwendung von Verbindungen der Formel (I), in welcher
R¹ für gegebenenfalls durch Fluor ein- oder zweifach substituiertes tert.-Butyl oder Cyclopropyl steht,
R² für Wasserstoff, Methyl oder Ethyl steht,
A für C-R³ steht, wobei
R³ für Wasserstoff, Methoxy, Difluormethoxy oder Cyano steht oder auch gemeinsam mit R¹ eine Brücke der Struktur -*O-CH₂-CH-CH₃ oder-*O-CH₂-N-CH₃, wobei das mit * markierte Atom mit dem Kohlenstoffatom von A verbunden ist, bilden kann,
R⁴ für Wasserstoff oder Methyl steht,
R⁶ für Wasserstoff steht,
von deren pharmazeutisch verwendbaren Hydraten und/oder Salzen zur Herstellung von Arzneimitteln zur Therapie von Helicobacter-pylori-Infektionen und den damit assoziierten gastroduodenalen Erkrankungen.

4. Verwendung von diastereomerenreinen und enantiomerenreinen Verbindungen gemäß den Ansprüchen 1 bis 3 von deren pharmazeutisch verwendbaren Hydraten und/oder Salzen zur Herstellung von Arzneimitteln zur Therapie von Helicobacter-pylori-Infektionen und den damit assoziierten gastroduodenalen Erkrankungen.

5. Verwendung von 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure von deren pharmazeutisch verwendbaren Hydraten und/oder Salzen zur Herstellung von Arzneimitteln zur Therapie von Helicobacter-pylori-Infektionen und den damit assoziierten gastroduodenalen Erkrankungen.

6. Diastereomerenreine und enantiomerenreine Verbindungen aus der Gruppe bestehend aus
8-Cyano-1-cyclopropyl-6-fluor-7-(2-oxa-5,8-diazabicyclo[4.3.0]non-8-yl)-1,4-dihydro-4-oxo-3-chinolincarbonsäure,
1-Cyclopropyl-8-difluormethoxy-6-fluor-1,4-dihydro-7-(2-oxa-5,8-diazabicyclo[4.3.0]non-8-yl)-4-oxo-3-chinolincarbonsäure und deren pharmazeutisch verwendbare Hydrate und/oder Salze.

7. 8-Cyano-1-cyclopropyl-6-fluor-7-((1S,6S)-2-oxa-5,8-diazabicyclo-[4.3.0]non-8-yl)-1,4-dihydro-4-oxo-3-chinolincarbonsäure, deren pharmazeutisch verwendbaren Hydrate und/oder Salze.

8. Verwendung von 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure, von deren pharmazeutisch verwendbaren Hydraten und/oder Salzen zur Herstellung von Arzneimitteln zur Therapie von Helicobacter-pylori-Infektionen und den damit assoziierten gastroduodenalen Erkrankungen.

9. Arzneimittel enthaltend 8-Cyano-1-cyclopropyl-6-fluor-7-((1S,6S)-2-oxa-5,8-diazabicyclo[4.3.0]non-8-yl)-1,4-dihydro-4-oxo-3-chinolincarbonsäure, deren pharmazeutisch verwendbaren Hydrate und/oder Salze.

## Claims

1. Use of compounds of the general formula (I) 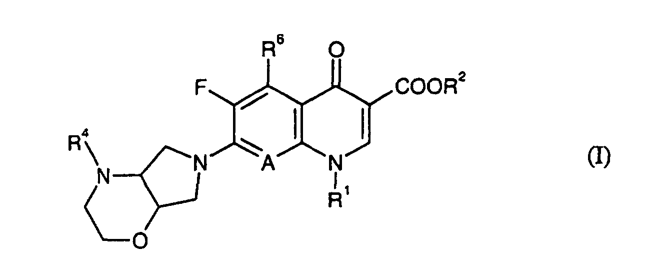 in which
R¹ represents alkyl having 1 to 4 C atoms, which is optionally mono- or disubstituted by halogen, phenyl which is optionally substituted by 1 or 2 fluorine atoms or cyclopropyl which is optionally substituted by 1 or 2 fluorine atoms,
R² represents hydrogen, alkyl having 1 to 4 carbon atoms, which is optionally substituted by hydroxyl, methoxy, amino, methylamino or dimethylamino, or (5-methyl-2-oxo-1,3-dioxol-4-yl)-methyl,
A represents N or C-R³, where
R³ represents hydrogen, halogen, methyl, methoxy, difluoromethoxy or cyano alternatively, together with R¹, can form a bridge of the structure -*O-CH₂-CH-CH₃ or -*O-CH₂- N-CH₃, where the atom marked by * is connected to the carbon atom of A,
R⁴ represents hydrogen, benzyl, C₁-C₃-alkyl, (5-methyl-2-oxo-1,3-dioxol-4-yl)-methyl, radicals of the structures -CH=CH-COOR⁵, -CH₂CH₂COOR⁵, -CH₂CH₂CN, -CH₂CH₂COCH₃, -CH₂COCH₃, in which
R⁵ represents methyl or ethyl,
R⁶ represents hydrogen, amino, hydroxyl, methyl or halogen,
in the form of racemates, diastereomer mixtures or as enantiomerically pure or diastereomerically pure compounds, and of their pharmaceutically utilizable hydrates and/or salts for the production of medicaments for the therapy of Helicobacter pylori infections and the gastroduodenal disorders associated therewith.

2. Use of compounds of the formula (I), in which
R¹ represents tert-butyl which is optionally mono- or disubstituted by fluorine or cyclopropyl which is optionally substituted by 1 fluorine atom,
R² represents hydrogen, alkyl having 1 to 4 carbon atoms or (5-methyl-2-oxo-1,3-dioxol-4-yl)-methyl,
A represents C-R³, where
R³ represents hydrogen, fluorine, methoxy, difluoromethoxy or cyano or alternatively, together with R¹, can form a bridge of the structure -*O-CH₂-CH-CH₃ or -*O-CH₂-N-CH₃, where the atom marked by * is connected to the carbon atom of A,
R⁴ represents hydrogen, C₁-C₃-alkyl, radicals of the structures, -CH₂CH₂COOR⁵, -CH₂CH₂CN, -CH₂COCH₃, in which
R⁵ represents methyl or ethyl,
R⁶ represents hydrogen, amino or methyl,
and their pharmaceutically utilizable hydrates and/or salts for the production of medicaments for the therapy of Helicobacter pylori infections and the gastroduodenal disorders associated therewith.

3. Use of compounds of the formula (I), in which
R¹ represents tert-butyl which is optionally mono- or disubstituted by fluorine, or cyclopropyl,
R² represents hydrogen, methyl or ethyl,
A represents C-R³, where
R³ represents hydrogen, methoxy, difluoromethoxy or cyano or alternatively, together with R¹, can form a bridge of the structure -*O-CH₂-CH-CH₃ or -*O-CH₂-N-CH₃, where the atom marked by * is connected to the carbon atom of A,
R⁴ represents hydrogen or methyl,
R⁶ represents hydrogen,
and of their pharmaceutically utilizable hydrates and/or salts for the production of medicaments for the therapy of Helicobacter pylori infections and the gastroduodenal disorders associated therewith.

4. Use of diastereomerically pure and enantiomerically pure compounds according to Claims 1 to 3 and of their pharmaceutically utilizable hydrates and/or salts for the production of medicaments for the therapy of Helicobacter pylori infections and the gastroduodenal disorders associated therewith.

5. Use of 8-cyano-1-cyclopropyl-6-fluoro-7-((1S,6S)-2-oxa-5,8-diazabicyclo-[4.3.0]non-8-yl)-1,4-dihydro-4-oxo-3-quinolinecarboxylic acid and of its pharmaceutically utilizable hydrates and/or salts for the production of medicaments for the therapy of Helicobacter pylori infections and the gastroduodenal disorders associated therewith.

6. Diastereomerically pure and enantiomerically pure compounds from the group consisting of
8-cyano- 1-cyclopropyl-6-fluoro-7-(2-oxa-5,8-di azabicyclo[4.3.0]non-8-yl)-1,4-dihydro-4-oxo-3-quinolinecarboxylic acid,
1-cyclopropyl-8-difluoromethoxy-6-fluoro-1,4-dihydro-7-(2-oxa-5,8-diazabicyclo[4.3.0]non-8-yl)-4-oxo-3-quinolinecarboxylic acid and their pharmaceutically utilizable hydrates and/or salts.

7. 8-Cyano-1-cyclopropyl-6-fluoro-7-((1S,6S)-2-oxa-5,8-diazabicyclo[4.3.0]-non-8-yl)-1,4-dihydro-4-oxo-3-quinolinecarboxylic acid and its pharmaceutically utilizable hydrates and/or salts.

8. Use of 8-cyano-1-cyclopropyl-6-fluoro-7-((lS,6S)-2-oxa-5,8-diazabicyclo-[4.3.0]non-8-yl)-1,4-dihydro-4-oxo-3-quinolinecarboxylic acid and its pharmaceutically utilizable hydrates and/or salts for the production of medicaments for the therapy of Helicobacter pylori infections and the gastroduodenal disorders associated therewith.

9. Medicament comprising 8-cyano-1-cyclopropyl-6-fluoro-7-((1S,6S)-2-oxa-5,8-diazabicyclo[4.3.0]non-8-yl)-1,4-dihydro-4-oxo-3-quinolinecarboxylic acid and its pharmaceutically utilizable hydrates and/or salts.

## Revendications

1. Utilisation de composés de formule générale (I) 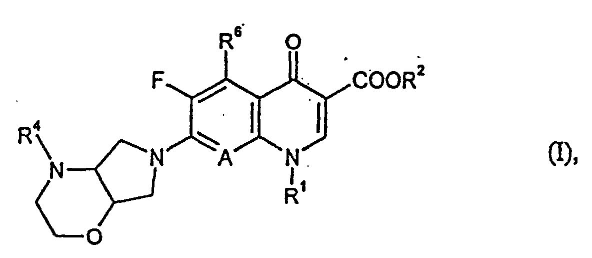 dans laquelle
R¹ est un groupe alkyle ayant 1 à 4 atomes de carbone, éventuellement substitué une ou deux fois par un halogène, un groupe phényle éventuellement substitué par 1 ou 2 atomes de fluor ou un groupe cyclopropyle éventuellement substitué par 1 ou 2 atomes de fluor,
R² représente l'hydrogène, un groupe alkyle ayant 1 à 4 atomes de carbone éventuellement substitué par un radical hydroxy, méthoxy, amino, méthylamino ou diméthylamino, ou le groupe (5-méthyl-2-oxo-1,3-dioxole-4-yl)-méthyle,
A représente N ou un groupe C-R³, dans lequel
R³ est l'hydrogène, un halogène, un groupe méthyle, méthoxy, difluorométhoxy ou cyano ou peut aussi former conjointement avec R¹ un pont de structure -*O-CH₂-CH-CH₃ ou -*O-CH₂-N-CH₃, dans lequel l'atome marqué d'un astérisque est lié à l'atome de carbone de A,
R⁴ représente l'hydrogène, un groupe benzyle, alkyle en C₁ à C₃, (5-méthyl-2-oxo-1,3-dioxole-4-yl)-méthyle, des restes de structures -CH=CH-COOR⁵, -CH₂CH₂COOR⁵, -CH₂CH₂CN, -CH₂CH₂COCH₃, -CH₂COCH₃, dans lesquels
R⁵ représente le groupe méthyle ou éthyle,
R⁶ est l'hydrogène, un groupe amino, hydroxy, méthyle ou un halogène,
sous forme de racémates, de mélanges de diastéréo-isomères ou comme composés énantiomères purs ou diastéréo-isomères purs, de leurs hydrates et/ou de leurs sels acceptables du point de vue pharmaceutique, pour l'obtention de médicaments destinés au traitement d'infections à Helicobacter pylori et des maladies gastroduodénales qui y sont associées.

2. Utilisation de composés de formule (I), dans laquelle
R¹ est un groupe tertio-butyle éventuellement substitué une ou deux fois par du fluor ou un groupe cyclopropyle éventuellement substitué par 1 atome de fluor,
R² représente l'hydrogène, un groupe alkyle ayant 1 à 4 atomes de carbone ou le groupe (5-méthyl-2-oxo-1,3-dioxole-4-yl)-méthyle,
A représente un groupe C-R³, dans lequel
R³ est l'hydrogène, le fluor, un groupe méthoxy, difluorométhoxy ou cyano, ou peut aussi former conjointement avec R¹ un pont de structure -*O-CH₂-CH-CH₃ ou -*O-CH₂-N-CH₃, dans lequel l'atome marqué d'un astérisque est lié à l'atome de carbone de A,
R⁴ représente l'hydrogène, un groupe alkyle en C₁ à C₃, des restes de structures -CH₂CH₂COOR⁵, -CH₂CH₂CN, -CH₂COCH₃, dans lesquels
R⁵ représente le groupe méthyle ou éthyle,
R⁶ est l'hydrogène, un groupe amino ou méthyle,
de leurs hydrates et/ou de leurs sels acceptables du point de vue pharmaceutique, pour l'obtention de médicaments destinés au traitement d'infections à Helicobacter pylori et des maladies gastroduodénales qui y sont associées.

3. Utilisation de composés de formule (I), dans laquelle
R¹ est un groupe tertio-butyle éventuellement substitué une ou deux fois par du fluor ou un groupe cyclopropyle,
R² représente l'hydrogène, le groupe méthyle ou éthyle,
A est un groupe C-R³, dans lequel
R³ représente l'hydrogène, un groupe méthoxy, difluorométhoxy ou cyano, ou peut aussi former conjointement avec R¹ un pont de structure -*O-CH₂-CH-CH₃ ou -*O-CH₂-N-CH₃, dans lequel l'atome marqué d'un astérisque est lié à l'atome de carbone de A,
R⁴ est l'hydrogène ou le groupe méthyle,
R⁶ est l'hydrogène,
de leurs hydrates et/ou de leurs sels acceptables du point de vue pharmaceutique, pour l'obtention de médicaments destinés au traitement d'infections à Helicobacter pylori et des maladies gastroduodénales qui y sont associées.

4. Utilisation de composés diastéréo-isomères purs ou énantiomères purs suivant les revendications 1 à 3, de leurs hydrates et/ou de leurs sels acceptables du point de vue pharmaceutique, pour l'obtention de médicaments destinés au traitement d'infections à Helicobacter pylori et des maladies gastroduodénales qui y sont associées.

5. Utilisation de l'acide 8-cyano-1-cyclopropyl-6-fluoro-7-((1S,6S)-2-oxa-5,8-diazabicyclo[4.3.0]non-8-yl)-1,4-dihydro-4-oxo-3-quinoléinecarboxylique, de ses hydrates et/ou de ses sels acceptables du point de vue pharmaceutique pour l'obtention de médicaments destinés au traitement d'infections à Helicobacter pylori et des maladies gastroduodénales qui y sont associées.

6. Composés diastéréo-isomères purs et énantiomères purs du groupe consistant en
l'acide 8-cyano-1-cyclopropyl-6-fluoro-7-(2-oxa-5,8-diazabicyclo[4.3.0]non-8-yl)-1,4-dihydro-4-oxo-3-quinoléinecarboxylique,
l'acide 1-cyclopropyl-8-difluorométhoxy-6-fluoro-1,4-dihydro-7-(2-oxa-5,8-diazabicyclo[4.3.0]non-8-yl)-4-oxo-3-quinoléinecarboxylique et leurs hydrates et/ou leurs sels acceptables du point de vue pharmaceutique.

7. L'acide 8-cyano-1-cyclopropyl-6-fluoro-7-((1S,6S)-2-oxa-5,8-diazabicyclo[4.3.0]non-8-yl)-1,4-dihydro-4-oxo-3-quinoléinecarboxylique, ses hydrates et/ou ses sels acceptables du point de vue pharmaceutique.

8. Utilisation de l'acide 8-cyano-1-cyclopropyl-6-fluoro-7-((1S,6S)-2-oxa-5,8-diazabicyclo[4.3.0]non-8-yl)-1,4-dihydro-4-oxo-3-quinoléinecarboxylique, de ses hydrates et/ou de ses sels acceptables du point de vue-pharmaceutique pour l'obtention de médicaments destinés au traitement d'infections à Helicobacter pylori et des maladies gastroduodénales qui y sont associées.

9. Médicament contenant de l'acide 8-cyano-1-cyclopropyl-6-fluoro-7-((1S,6S)-2-oxa-5,8-diazabicyclo-[4.3.0] non-8-yl)-1,4-dihydro-4-oxo-3-quinoléine-carboxylique, ses hydrates et/ou ses sels acceptables du point de vue pharmaceutique.
